(19) Europäisches Patentamt
European Patent Office
Office européen des brevets

(11) Veröffentlichungsnummer: **0 206 197 A1**

(12) # EUROPÄISCHE PATENTANMELDUNG

(21) Anmeldenummer: **86108186.7**

(22) Anmeldetag: **18.06.86**

(51) Int. Cl.⁴: **C 07 D 417/04**, C 07 D 277/06, A 61 K 31/425

(30) Priorität: **18.06.85 HU 238885**

(43) Veröffentlichungstag der Anmeldung: **30.12.86 Patentblatt 86/52**

(84) Benannte Vertragsstaaten: **AT BE CH DE FR IT LI LU NL SE**

(71) Anmelder: **BIOGAL GYOGYSZERGYAR, Pallagi u. 13., H-4042 Debrecen (HU)**

(72) Erfinder: **Györgydeák, Zoltán, Dr., Nagyerdei krt. 74.II.6, Debrecen (HU)**
Erfinder: **Kovács, István, Dr., Csapó u. 86.II.6, Debrecen (HU)**
Erfinder: **Bognár, Rezsö, Dr., Dóczy J. u. 7, Debrecen (HU)**
Erfinder: **Horváth, Géza, Dr., Gyergyó u. 9.X.59, Debrecen (HU)**

(72) Erfinder: **Mile, Terézia, Dr., Monostor u. 2, Debrecen (HU)**
Erfinder: **Krusper geb. Hám, Judit, Gáborjáni Szabó u. 21, Debrecen (HU)**
Erfinder: **Pusztai, Ferenc, Dr., Laktanya u. 6, Debrecen (HU)**
Erfinder: **Fekete geb. Huszka, Mariann, Szabó I.alt.tér 8, Debrecen (HU)**
Erfinder: **Jancsó, Sándor, Dr., Kardos u. 28, Debrecen (HU)**
Erfinder: **Bálint, János, Dr., Péchy u. 5, Debrecen (HU)**
Erfinder: **Mihók geb. Borbély, Ildikó, Dr., Martonfalvi u. 7, Debrecen (HU)**
Erfinder: **Jakab, Attila, Sinai M. u. 9, Debrecen (HU)**
Erfinder: **Jenei, András, Dr., Naphegy u. 57, Budapest (HU)**
Erfinder: **Szende, Béla, Dr., Ollöi u. 55, Budapest (HU)**
Erfinder: **Lapis, Károly, Dr., Szamuely u. 25, Budapest (HU)**

(74) Vertreter: **Beszédes, Stephan G. Dr., Münchener Strasse 80a Postfach 1168, D-8060 Dachau (DE)**

(54) **5,5-Di-(methyl)-thiazolidin-4(S)-carbonsäurederivate, Verfahren zu ihrer Herstellung und diese Verbindungen enthaltende Arzneimittel.**

(57) Gegenstand der Erfindung sind 5,5-Di-(methyl)-thiazolidin-4(S)-carbonsäurederivate der allgemeinen Formel

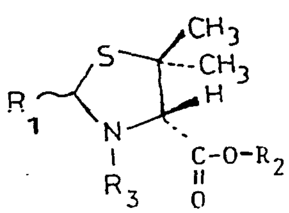

I,

worin

$R_1$ für einen, gegebenenfalls substituierten, Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest oder einen, gegebenenfalls durch eine Carboxy- oder Hydroxylgruppe substituierten, Alkylrest mit 1 bis 4 Kohlenstoffatom(en) oder Alkenylrest mit 2 bis 4 Kohlenstoffatomen

$R_2$ Wasserstoff, ein Alkalimetall- oder Erdalkalimetallatom, einen, gegebenenfalls substituierten, Alkylrest mit 1 bis 4 Kohlenstoffatom(en) oder einen, gegebenenfalls substituierten, Arylrest bedeutet und

$R_3$ Wasserstoff, einen gegebenenfalls substituierten, Alkyl- oder Acylrest mit 1 bis 4 Kohlenstoffatom(en) oder einen, gegebenenfalls substituierten, Arylrest darstellt

sowie ihre Salze.

Ferner sind Gegenstand der Erfindung ein Verfahren zur Herstellung dieser Verbindungen und sie enthaltende Arzneimittel, insbesondere mit Leberschutzwirkung.

EP 0 206 197 A1

Die Erfindung betrifft neue 5,5-Di-(methyl)--thiazolidin-4(S)-carbonsäurederivate, ein Verfahren zu ihrer Herstellung und diese Verbindungen enthaltende Arzneimittel, insbesondere mit Leberschutzwirkung.

Es wurden bereits biologisch wirksame Thiazolidin--4-carbonsäuren beschrieben, unter diesen auch solche mit Leberschutzwirkung, zum Beispiel die in der französischen Patentschrift 923 562 erwähnten Thiazolidin-4-carbonsäuren, die eine Leberschutzwirkung, leberregenerierende, antinekrotische und proteinotrope Wirkung haben sollen.

Zur Herstellung von Thiazolidin-4-carbonsäure-derivaten ist in der DE-PS 2 116 629 ein Verfahren, gemäß welchem Cysteinhydrochlorid mit Brenztraubensäure /zur Herstellung von 2-(Methyl)-thiazolidin-2,4-dicarbonsäure/ oder mit m-(Formyl)-phenoxyessigsäure /zur Herstellung von 2-(2'-{Phenoxy}-acetyl)-thiazolidin-4-carbonsäure/ oder sonstigen organischen Säuren beschrieben. Die genannten Verbindungen haben eine Leberschutzwirkung.

Antihyperlipämisch wirkt die in der DE-PS 2 446 100 beschriebene 5,5-Di-(methyl)-3-/p-(chlorphenoxy)-isobutyryl/-thiazolidin-4-carbonsäure.

In der DE-PS 2 614 798 sind 2-/Methylamino/-5-/5'--(nitro)-furfur-2'-yliden)-thiazolin-4-on und 3-/Methyl/--2-/imino/-5-/5'-(nitro)-furfur-2'-yliden/-thiazolidin--4-on, die bei der Papierherstellung Verwendung finden, beschrieben.

Die belgische Patentschrift Nr. 751 482 betrifft Thiazolidin-2,4-di-(carbonsäuren) und ihre Derivate. Die Verbindungen wirken leberschützend, leberregenerierend

und detoxizierend. Die Herstellung der Thiazolidin-2,4-di-(carbonsäuren) geht von Cystein aus und wird mit Natriumacetat in Gegenwart von Triäthylamin vorgenommen.

Eine Veröffentlichung von F. Asinger und Mitarbeitern (Monatshefte für Chemie 114 [1983], 47 bis 63) betrifft die Herstellung von 5,5-Di-(methyl)-thiazolidin-4(R,S)-carbonsäuren und ihrer Derivate. Gemäß der Mitteilung wird D,L-Penicillamin-hydrochlorid mit verschiedenen Aldehyden und Ketonen kondensiert. Die Endprodukte haben zwei Chiralitätszentren und fallen als Racemate in Form des Hydrochlorids oder der freien Base an. Die biologische Wirkung der Verbindungen wurde nicht untersucht, weil der Gegenstand der Versuche der Mechanismus der Kondensationsreaktion war.

Penicillamin [ß,ß-Di-(methyl)-cystein] ist eine mehrere funktionelle Gruppen aufweisende Aminosäure, deren eine charakteristische Reaktion es ist, mit Carbonylgruppen enthaltenden Verbindungen zu einem Ringschluß führende Kondensationsreaktionen einzugehen. Durch diese Reaktion entstehen in der 2-Stellung verschiedene Substituenten aufweisende 5,5-Di-(methyl)-thiazolidin-4-carbonsäuren, aus denen durch weitere chemische Reaktionen Ester, Amide, Schwefel- und Stickstoffderivate erhältlich sind.

Wie es aus dem Obigen hervorgeht, ist für einen Teil der bekannten Thiazolidincarbonsäure-derivate, nicht jedoch die 5,5-Di-(methyl)-thiazolidin-4(R,S)-carbonsäuren und ihre Derivate, eine Leberschutzwirkung bekannt.

Diese Wirkung beruht nach den gegenwärtigen Erkenntnissen darauf, daß die Verbindungen an dem Vorgang der Reaktivierung von Leberenzymen und anderen körpereigenen Enzymen teilnehmen; bei diesem Vorgang werden die überhandnehmenden Disulfidgruppen erneut zu freien Thiolgruppen umgewandelt.

Es sind Versuche bekannt, die Thiazolidin-4-carbonsäuren von D,L-Cystein oder D,L-Penicillamin ausgehend herzustellen. Bei beiden Reaktionstypen entstehen racemische Verbindungen mit 2 Asymmetriezentren, das heißt je Reakton 4 Isomere mit unterschiedlicher Raumstruktur. Die Moleküle unterschiedlicher Raumstruktur haben verschiedene biologische Aktivitäten, es kommen unter ihnen auch inaktive Formen vor.

Um die inaktiven oder wenig aktiven Formen in Kenntnis ihrer Aktivität von vornherein auszusondern, wurde nun die Möglichkeit in Betracht gezogen, als Ausgangsverbindung immer nur das eine optische Isomer einzusetzen, weil dadurch im Endprodukt das eine Asymmetriezentrum fixiert ist und die Anzahl der möglichen Isomere auf die Hälfte sinkt.

Der Erfindung liegt die Aufgabe zugrunde, überlegene pharmakologische Wirkungen, insbesondere eine Leberschutzwirkung, aufweisende neue 5,5-Di-(methyl)-thiazolidin-4(S)-carbonsäurederivate, ein Verfahren zu ihrer Herstellung und diese Verbindungen enthaltende Arzneimittel zu schaffen.

Das Obige wurde überraschenderweise durch die Erfindung erreicht.

In Anbetracht des Obigen sind erfindungsgemäß als neue pharmakologisch wirksame Verbindungen von den bei der Kondensation entstehenden unterschiedlichen räumlichen Isomeren der Thiazolidin-4-carbonsäurederivate bestimmte von besonderem Interesse.

Gegenstand der Erfindung sind daher 5,5-Di-(methyl)-thiazolidin-4(S)-carbonsäurederivate der allgemeinen Formel

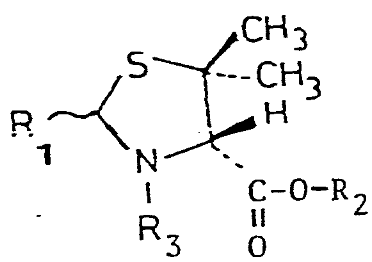

I ,

worin

$R_1$ für einen, gegebenenfalls substituierten, Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest oder einen, gegebenenfalls durch eine Carboxy- oder Hydroxylgruppe substituierten, Alkylrest mit 1 bis 4 Kohlenstoffatom(en) oder Alkenylrest mit 2 bis 4 Kohlenstoffatomen steht,

$R_2$ Wasserstoff, ein Alkalimetall- oder Erdalkalimetallatom, einen, gegebenenfalls substituierten, Alkylrest mit 1 bis 4 Kohlenstoffatom(en) oder einen, gegebenenfalls substituierten, Arylrest bedeutet und

$R_3$ Wasserstoff, einen gegebenenfalls substituierten, Alkyl- oder Acylrest mit 1 bis 4 Kohlenstoffatom(en) oder einen, gegebenenfalls substituierten, Arylrest darstellt

sowie ihre Salze.

Vorteilhaft sind die 5,5-Di-(methyl)-thiazolidin-4(S)-carbonsäurederivate der allgemeinen Formel I solche, in welchen

$R_1$ für einen, gegebenenfalls durch 1 oder mehr Alkylrest(e) mit 1 bis 4 Kohlenstoffatom(en), Nitrogruppe(n),

Aminogruppe(n), Halogenatom(e), Hydroxygruppe(n), Hydroxyalkylrest(e) mit 1 bis 4 Kohlenstofatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n] Alkylcarbonylrest(e) mit 2 bis 4 Kohlenstoffatomen substituierten, Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest oder einen, gegebenenfalls durch eine Carboxyl- oder Hydroxygruppe substituierten, Alkylrest mit 1 bis 4 Kohlenstoffatom(en) oder Alkenylrest mit 2 bis 4 Kohlenstoffatomen steht,

$R_2$ Wasserstoff, ein Alkalimetall oder Erdalkalimetall, einen, gegebenenfalls durch 1 oder mehr Nitrogruppe(n), Aminogruppe(n), Halogenatome(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder

Formyl- und/oder, gegebenenfalls durch 1 oder mehr Hydroxygruppe(n) substituierte[n], Alkylcarbonylrest(e) mit 2 bis 4 Kohlenstoffatomen substituierten, Alkylrest mit 1 bis 4 Kohlenstoffatom(en) oder, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen substituierten, Arylrest bedeutet und

$R_3$ Wasserstoff einen, gegebenenfalls durch 1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4

Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen substituierten, Alkylrest mit 1 bis 4 Kohlenstoffatom(en), einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Alkylthiorest(e) mit 1 bis 4 Kohlenstoffatom(en) und/oder, gegebenenfalls durch 1 oder mehr Halogenatom(e) substituierte/n/ Phenoxyrest(e) substituierten, Formyl- beziehungsweise Alkylcarbonylrest mit 2 bis 4 Kohlenstoffatomen oder einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr

Hydroxygruppe(n) substituierte[n], Alkylcarbonylrest(e) mit 2 bis 4 Kohlenstoffatomen substituierten, Arylrest darstellt.

Vorzugsweise ist beziehungsweise sind der Alkylrest, für den $R_1$ stehen kann oder der beziehungsweise die Alkylrest(e), Hydroxyalkylrest(e), Alkoxyrest(e) und/oder Alkylthiorest(e), durch welche[n] der Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest, für den $R_1$ stehen kann, substituiert sein kann, und/oder der beziehungsweise die Hydroxyalkylrest(e), Alkoxyrest(e) und/oder Alkylthiorest(e), durch welche[n] der Alkyl- oder Arylrest, für den $R_2$ stehen kann, substituiert sein kann, oder der beziehungsweise die Alkylrest(e), durch welche[n] der Arylrest, für den $R_2$ stehen kann, substituiert sein kann, und/oder der beziehungsweise die Hydroxyalkylrest(e), Alkoxyrest(e) und/oder Alkylthiorest(e), durch welche[n] der Alkylrest oder Formyl- beziehungsweise Alkylcarbonylrest, für den $R_3$ stehen kann, substituiert sein kann, oder der beziehungsweise die Alkylrest(e), durch welche[n] der Formyl- beziehungsweise Alkylcarbonylrest oder Arylrest, für den $R_3$ stehen kann, substituiert sein kann, [ein] solche[r] mit 1 oder 2, insbesondere 1, Kohlenstoffatom(en).

Es ist auch bevorzugt, daß der Alkenylrest, für den $R_1$ stehen kann und/oder der beziehungsweise die Alkylcarbonyloxyrest(e) und/oder Alkylcarbonylrest(e), durch welche[n] der Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest substituiert sein kann, und/oder der beziehungsweise die Alkylcarbonyloxyrest(e), durch welche[n] der Alkyl- oder Arylrest, für den $R_2$ stehen kann, substituiert sein kann und/oder der beziehungsweise die Alkylcarbonyloxyrest(e), durch welche[n] der Alkyl- oder Arylrest, für den $R_3$ stehen kann, substituiert sein kann, und/oder der Alkylcarbonylrest, für den $R_3$ stehen kann, [ein] solche[r] mit 2 oder 3, insbesondere 2, Kohlenstoffatomen ist beziehungsweise sind.

Ferner ist es bevorzugt, daß der beziehungsweise die Arylrest(e), für den beziehungsweise die $R_2$ und/oder $R_3$ stehen kann beziehungsweise können, [ein] Phenylrest(e) ist beziehungsweise sind.

Weiterhin ist es bevorzugt, daß das beziehungsweise die Halogenatom(e), durch welche/s/ der Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest, für den $R_1$ stehen kann, der Alkyl- oder Arylrest, für den $R_2$ stehen kann, und/oder der Alkylrest, Formyl- beziehungsweise Alkylcarbonylrest oder Arylrest, für den $R_3$ stehen kann, substituiet sein kann, und/oder das beziehungsweise die Halogenatom(e), durch welche[s] der beziehungsweise die Phenoxyrest(e), durch welche[n] der Formyl-beziehungsweise Alkylcarbonylrest, für den $R_3$ stehen kann, substituiert sein kann beziehungsweise können, substituiert sein kann, Chlor, Brom und/oder Fluor ist beziehungsweise sind.

Sofern der Pyrryl- beziehungsweise Benzopyrrylrest, für den $R_1$ stehen kann, substituiert ist, ist er vorzugsweise an seinem Stickstoffatom substituiert. Dabei ist es auch bevorzugt, daß der Substituent ein Alkylrest mit 1 bis 4 Kohlenstoffatom(en) ist.

Vorzugsweise ist das Alkalimetall, für das $R_2$ stehen kann, Kalium und das Erdalkalimetall, für das $R_2$ stehen kann, Magnesium.

Beispiele für substituierte Reste, für die $R_1$ stehen kann, sind 5-(Nitro)-furyl-, 3-(Nitro)-phenyl-, N-(Methyl)-pyrryl-, 4-(Chlor)-phenyl-, 4-(Fluor)-phenyl-, (p-Methylthio)-phenyl-, 2-(Carboxy)-phenyl-, 3,4-Di-(methoxy)-2-(carboxy)-phenyl-, 2-(Hydroxy)-methyl-, 3,5-Di-(brom)-4-(hydroxy)-phenyl-, 2-(Methyl)-5-(hydroxymethyl)-3-(hydroxy)-pyridyl-, 2,4-Di-(chlor)-phenyl-, p-Tolyl-, 2- und 4-(Hydroxy)-phenyl- und 2- und 4-(Acetoxy)-phenylreste.

Bei den erfindungsgemäßen 5,5-Di-(methyl)-thiazolidin-4(S)-carbonsäurederivaten, bei welchen $R_1$ für einen 5-gliedrigen heterocyclischen Rest mit einem Heteroatom steht, bilden die der allgemeinen Formel

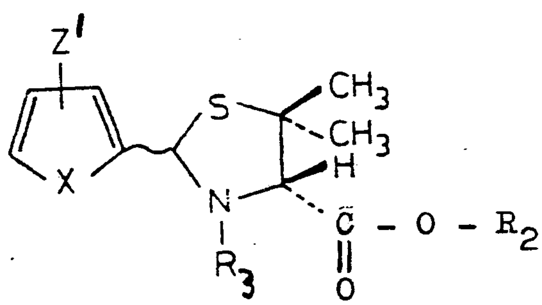

II ,

worin

X für Sauerstoff, Schwefel oder einen Rest der Formel $-\overset{R_0}{\overset{|}{N}}-$ mit $R_0$ = Wasserstoff oder

Alkylrest mit 1 bis 4 Kohlenstoffatom(en), Nitrogruppe, Aminogruppe, Halogenatom, Hydroxygruppe, Hydroxyalkylrest mit 1 bis 4 Kohlenstoffatom(en), Carboxygruppe, Alkoxyrest mit 1 bis 4 Kohlenstoffatom(en), Mercaptorest, Alkylthiorest mit 1 bis 4 Kohlenstoffatom(en), Formyloxy- oder Alkylcarbonyloxyrest mit 2 bis 4 Kohlenstoffatomen oder Formyl- oder, gegebenenfalls durch 1 oder mehr Hydroxygruppe(n) substituierte[r] Alkylcarbonylrest mit 2 bis 4 Kohlenstoffatomen steht, und

Z' Wasserstoff, einen Alkylrest mit 1 bis 4 Kohlenstoffatom(en), eine Nitrogruppe, eine Aminogruppe, ein Halogenatom, eine Hydroxygruppe, einen Hydroxyalkylrest mit 1 bis 4 Kohlenstoffatom(en), eine Carboxygruppe, einen Alkoxyrest mit 1 bis 4 Kohlenstoffatom(en), einen Mercaptorest oder einen Formyl- oder, gegebenenfalls durch 1 oder mehr Hydroxygruppe(n) substituierten Alkylcarbonylrest mit 2 bis 4 Kohlenstoffatomen.bedeutet,

eine Untergruppe.

Bei den erfindungsgemäßen 5,5-Di-(methyl)-thiazolidin--4(S)-carbonsäurederivate, bei welchen $R_1$ für einen aus kondensierten Ringen bestehenden Rest steht, bilden die der allgemeinen Formeln

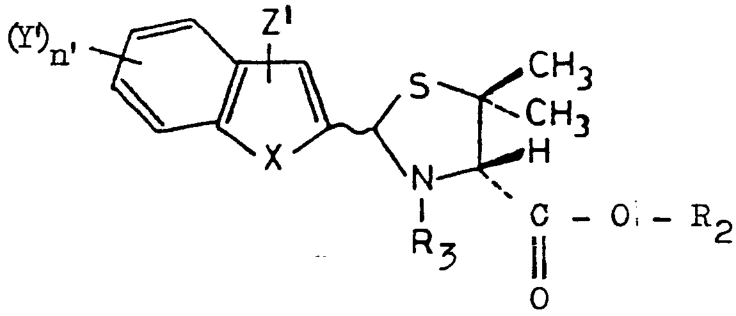

III,

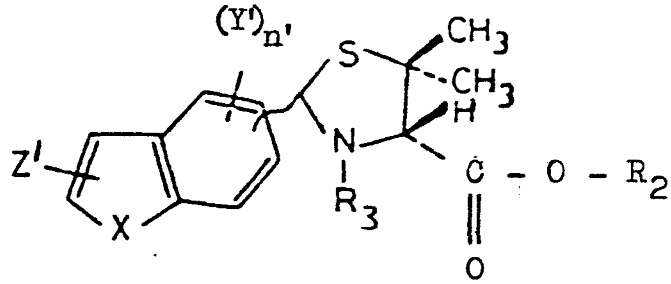

IV,

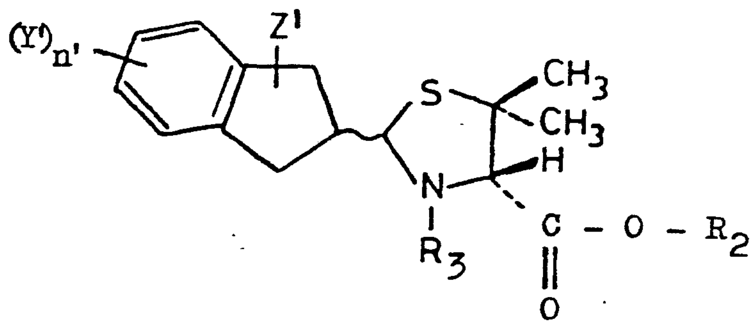

IIIa

beziehungsweise

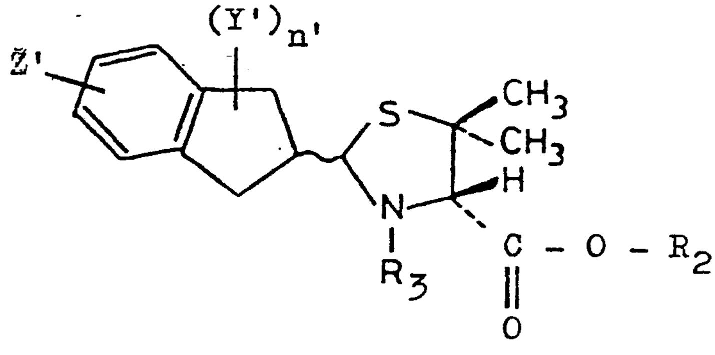

IVa ,

0206197

worin

Y für Wasserstoff, einen Alkylrest mit 1 bis 4 Kohlenstoffatom(en), eine Nitrogruppe, ein Halogenatom, eine Hydroxygruppe, einen Hydroxyalkylrest mit 1 bis 4 Kohlenstoffatom(en), eine Carboxygruppe, einen Mercaptorest, einen Formyloxy- beziehungsweise Alkylcarbonyloxyrest oder einen Formyl- beziehungsweise Alkylcarbonylrest steht und

n' eine ganze Zahl von 1 bis 3 darstellt,

eine Untergruppe.

Bei den erfindungsgemäßen 5,5-Di-(methyl)-thiazolidin-4(S)-carbonsäurederivaten bei welchen $R_1$ für einen Phenylrest beziehungsweise Pyridylrest steht, bilden die der allgemeinen Formeln

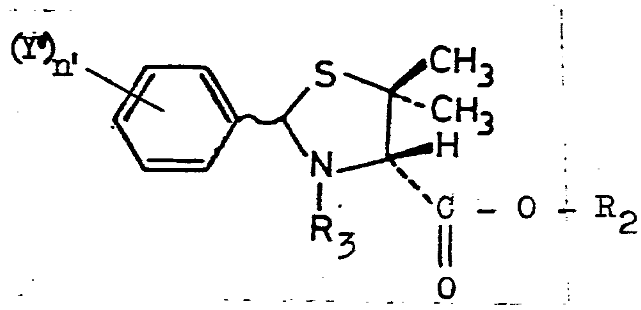

V

beziehungsweise

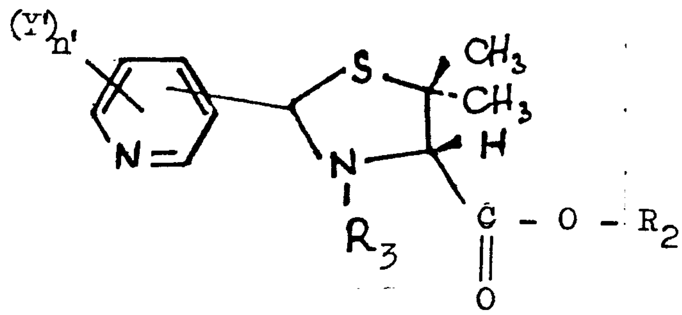

Va ,

worin Y' und n' die obigen Bedeutungen haben, eine Untergruppe.

Wenn $R_1$ für einen Pyridylrest steht, kann dieser in seiner 2-, 3- oder 4-Stellung an den 5,5-Di-(methyl)-thiazolidin-4(S)-carbonsäureteil gebunden sein, wie es die obige Formel Va zeigt.

Besonders bevorzugte erfindungsgemäße 5,5-Di-(methyl)-thiazolidin-4(S)-carbonsäurederivate sind 5,5-Di-[methyl]-2-[5'-{nitro}-fur-2'-yl]-thiazolidin-4(S)-carbonsäure, 5,5-Di-[methyl]-2-[fur-2'-yl]-thiazolidin-4(S)-carbonsäure, 5,5-Di-[methyl]-2-[thien-2'-yl]-thiazolidin-4(S)-carbonsäure, 5,5-Di-[methyl]-2-[N-(methyl)-pyrr-2'-yl]-thiazolidin-4(S)-carbonsäure, 5,5-Di-(methyl)-2-(pyrid-3'-yl)-thiazolidin-4(S)-carbonsäure, 5,5-Di-(methyl)-2-(pyrid-4'-yl)-thiazolidin-4(S)-carbonsäure, 5,5-Di-(methyl)-2-(benzofur-2'-yl)-thiazolidin-4(S)-carbonsäure, 5,5-Di-(methyl)-2-(indan-5'-yl)-thiazolidin-4(S)-carbonsäure, 5,5-Di-[methyl]-2-[3'-(nitro)-phenyl]-thiazolidin-4(S)-carbonsäure, 5,5-Di[methyl]-2-[4'-(fluor)-phenyl]-thiazolidin-4(S)-carbonsäure, 5,5-Di-[methyl]-2-[(p-

-methylthio)-phenyl]-thiazolidin-4(S)-carbonsäure, 5,5-Di-[methyl]-2-[2'-(carboxy)-phenyl]-thiazolidin-4(S)-carbonsäure, 5,5-Di-[methyl]-2-[3',4'-di-(methoxy)-2'-(carboxy)-phenyl]-thiazolidin-4(S)-carbonsäure, 5,5-Di-(methyl)-2-(hydroxymethyl)-thiazolidin-4(S)-carbonsäure, 5,5-Di-[methyl]-2-[3',5'-di-(brom)-4'-(hydroxy)-phenyl]-thiazolidin-4(S)-carbonsäure, 5,5-Di-[methyl]-2-[2'-(methyl)-5'-(hydroxymethyl)-3'-(hydroxy)-pyrid-4'-yl]-thiazolidin-4(S)-carbonsäure, 5,5-Di-[methyl]-2-[2',4'-di-(chlor)-phenyl]-thiazolidin-4(S)-carbonsäure, 5,5-Di-(methyl)-2-(p-tolyl)-thiazolidin-4(S)-carbonsäure, 5,5-Di-[methyl]-2-[4'-(hydroxy)-phenyl]-thiazolidin-4(S)-carbonsäure, 5,5-Di-[methyl]-2-[2'-(hydroxy)-phenyl]-thiazolidin-4(S)-carbonsäure, 5,5-Di-[methyl]-2(R)-[p-(fluor)-phenyl]-thiazolidin-4(S)-carbonsäure, 3-[Acetyl]-5,5-di-[methyl]-2(R)-[p-(chlor)-phenyl]-thiazolidin-4(S)-carbonsäure, 3-[Acetyl]-5,5-di-[methyl]-2(S)-[p-(chlor)-phenyl]-thiazolidin-4(S)-carbonsäure, 3-[Acetyl]-5,5-di-[methyl]-2(S)-[2'-(acetoxy)-phenyl]-thiazolidin-4(S)-carbonsäure, 3-[Acetyl]-5,5-di-[methyl]-2(S)-[2',4'-di-(chlor)-phenyl]-thiazolidin-4(S)-carbonsäure, 3-[Acetyl]-5,5-di-[methyl]-2(S)-[p-Tolyl]-thiazolidin-4(S)-carbonsäure, 3-[Acetyl]-5,5-di-[methyl]-2(S)-[phenyl]-thiazolidin-4(S)-carbonsäure, 3-[Acetyl]-5,5-di-[methyl]-2(S)-[4'-(acetoxy)-phenyl]-thiazolidin-4(S)-carbonsäure, 3-[Acetyl]-5,5-di-[methyl]-2(S)-[p-(fluor)-phenyl]-thiazolidin-4(S)-carbonsäure, 3-[Acetyl]-5,5-di-[methyl]-2(S)-[5'-(nitro)-fur-2'-yl]-thiazolidin-4(S)-carbonsäure, 3-[2'-{p-(chlorphenoxy)}-isobutyryl]-5,5-di-[methyl]-2(S)-[phenyl]-

-thiazolidin-4(S)-carbonsäure, 3-[Acetyl]-5,5-di-[methyl]-2(R)-[p-(fluor)-phenyl]-thiazolidin-4(S)-carbonsäure und 3-(Acetyl)-5,5-di-(methyl)-2(R)-(p-{chlor}phenyl)-thiazolidin-4(S)-carbonsäure.

Gegenstand der Erfindung ist auch ein Verfahren zur Herstellung der erfindungsgemäßen Verbindungen, welches dadurch gekennzeichnet ist, daß, gegebenenfalls an ihrem Stickstoffatom substituierte, ß,ß-Di-(methyl)-cysteine der allgemeinen Formel

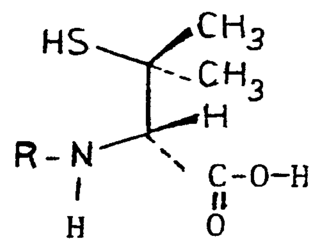

X ,

worin R für Wasserstoff, einen, gegebenenfalls, vorzugsweise durch 1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Alkylrest mit 1 bis 4 Kohlenstoffatom(en), einen, gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Alkylthiorest(e) mit 1 bis 4 Kohlenstoffatom(en) und/oder gegebenenfalls durch 1 oder mehr Halogenatom(e) substituierte[n], Phenoxyrest(e), substituierten, Formyl- beziehungsweise Alkylcarbonylrest mit 2 bis 4 Kohlenstoffatomen oder einen, gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n] Alkylcarbonylrest(e) mit 2 bis 4 Kohlenstoffatomen, substituierten, Arylrest, steht, mit Aldehyden der allgemeinen Formeln

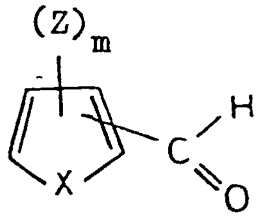 VI,

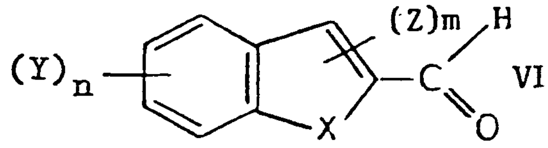 VII,

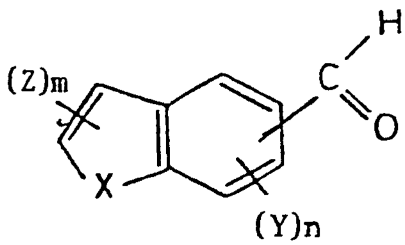 VIII,

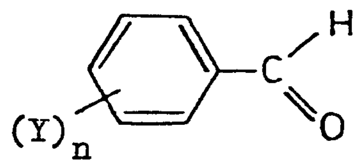 IX,

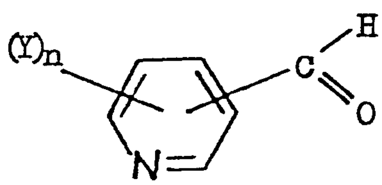 IXa,

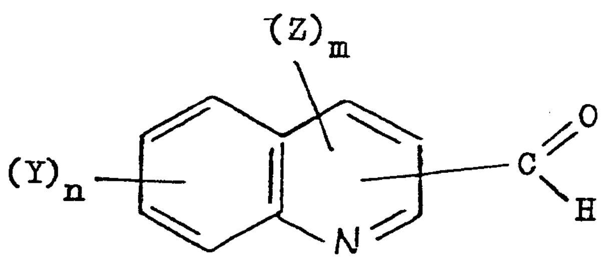 XI,

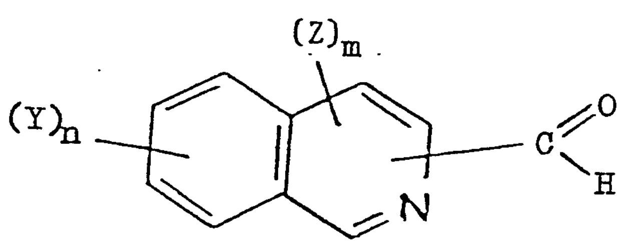 XIa,

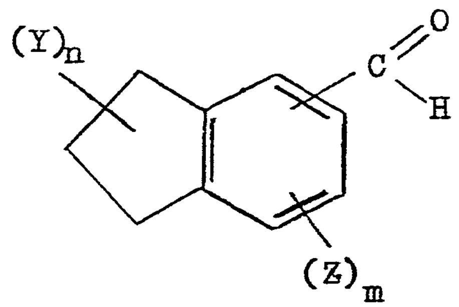

XII,

worin

X für Sauerstoff, Schwefel oder einen Rest der Formel $-\overset{R_0}{\overset{|}{N}}-$ mit $R_0$ = Wasserstoff oder einen organischen Rest, vorzugsweise Alkylrest mit 1 bis 4 Kohlenstoffatom(en), Nitrogruppe, Aminogruppe, Halogenatom, Hydroxygruppe, Hydroxyalkylrest mit 1 bis 4 Kohlenstoffatom(en), Carboxygruppe, Alkoxyrest mit 1 bis 4 Kohlenstoffatom(en), Mercaptorest, Alkylthiorest mit 1 bis 4 Kohlenstoffatom(en), Formyloxy- oder Alkylcarbonyloxyrest mit 2 bis 4 Kohlenstoffatomen oder

0206197

Formyl- oder, gegebenenfalls durch 1 oder mehr Hydroxygruppe(n) substituierte[n], Alkylcarbonylrest mit 2 bis 4 Kohlenstoffatomen, steht,

Z für gleiche oder verschiedene organische Rest(e), vorzugsweise Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formvl- und/oder gegebenenfalls durch 1 oder mehr Hydroxygruppe(n) substituierte[n], Alkylcarbonylrest(e) mit 2 bis 4 Kohlenstoffatomen, steht beziehungsweise stehen

Y gleiche oder verschiedene organische Rest(e), vorzugsweise Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit2 bis 4 Kohlenstoffatomen,

bedeutet beziehungsweise bedeuten und

n und m unabhängig voneinander ganze Zahlen von 0 bis 3 bedeuten,

oder mit Aldehyden der allgemeinen Formel

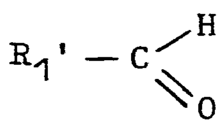

XIII ,

worin

$R_1'$ für einen, gegebenenfals durch eine Carboxyl- oder Hydroxygruppe substituierten, Alkylrest mit 1 bis 4 Kohlenstoffatom(en) oder Alkenylrest mit 2 bis 4 Kohlenstoffatomen steht,

umgesetzt werden sowie gegebenenfalls

a) diejenigen 5,5-Di-(methyl)-thiazolidin-4(S)--carbonsäurederivate der allgemeinen Formel I, bei welchen $R_2$ für Wasserstoff steht, in die entsprechenden Alkali- oder Erdalkalisalze überführt werden oder

b) diejenigen 5,5-Di-(methyl)-thiazolidin-4(S)--carbonsäurederivate der allgemeinen Formel I, bei welchen $R_2$ für Wasserstoff oder ein Alkali- oder Erdalkalimetall steht zu den entsprechenden 5,5-Di-(methyl)-thiazolidin-4(S)--carbonsäurederivaten der allgemeinen Formel I, bei welchen $R_2$ für einen, gegebenenfalls, vorzugsweise durch

1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Alkylrest mit 1 bis 4 Kohlenstoffatom(en) oder, gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Arylrest steht, verestert werden und/oder

c) diejenigen 5,5-Di-(methyl)-thiazolidin-4(S)--carbonsäurederivate der allgemeinen Formel I, bei welchen $R_3$ für Wasserstoff steht, mit Alkylhalogeniden oder -estern, Säureanhydriden

oder -halogeniden oder Arylhalogeniden oder -estern zu den entsprechenden 5,5-Di-(methyl)-thiazolidin-4(S)-carbonsäurederivaten der allgemeinen Formel I, bei welchen $R_3$ für einen, gegebenenfalls, vorzugsweise durch 1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Alkylrest mit 1 bis 4 Kohlenstoffatom(en), einen gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Alkylthiorest(e) mit 1 bis 4 Kohlenstoffatom(en) und/oder, gegebenenfalls, vorzugsweise durch 1 oder mehr Halogenatom(e) substituierte[n], Phenoxyrest(e) substituierten, Formyl- beziehungsweise Alkylcarbonylrest mit 2 bis 4 Kohlenstoffatomen oder einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en),

Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte/n/, Alkylcarbonylrest(e) mit 2 bis 4 Kohlenstoffatomen, substituierten, Arylrest steht, N-alkyliert, N-acyliert oder N-aryliert, wobei man gegebenenfalls gleichzeitig eine gegebenenfalls am Substituenten $R_1$ vorhandene Hydroxygruppe acyliert werden.

Die im erfindungsgemäßen Verfahren als Ausgangssubstanzen eingesetzten, gegebenenfalls an ihrem Stickstoffatom substituierten, ß,ß-Di-(methyl)-cysteine oder anders ausgedrückt Penicillamine der allgemeinen Formel X haben im Chiralitätszentrum D-Konfiguration. Erfindungsgemäß werden als Ausgangsstoffe im Gegensatz zu den bisherigen Verfahren D-Penicillamine eingesetzt. Dadurch sinkt die Anzahl der entstehenden Isomere auf die Hälfte, nur die cis-(2S/4S)- und die trans-(2R/4S)-Diastereomere treten auf.

Beispielhafte Gruppen der im erfindungsgemäßen Verfahren als Ausgangssubstanzen verwendbaren Aldehyde der allgemeinen Formeln VI, VII, VIII, XI, XIa und XII sind die der Formeln

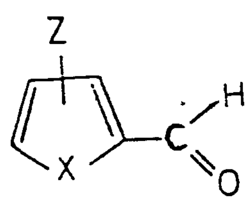 VI',

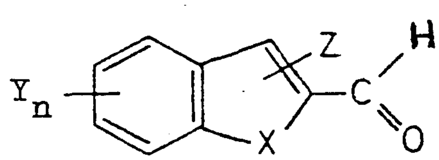 VII',

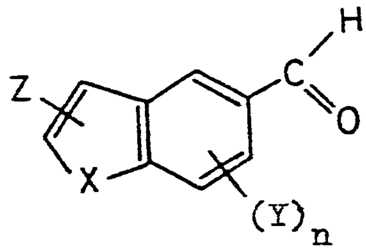 VIII',

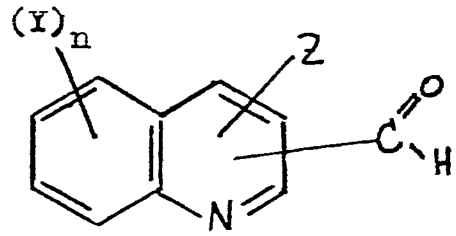 XI',

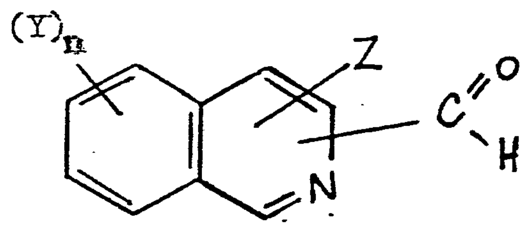 XIa',

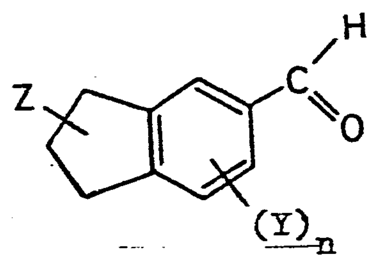 XII'

und

 0206197

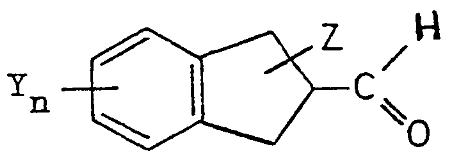

XII'',

worin X, Z, Y und n die obigen Bedeutungen haben.

Die nach dem erfindungsgemäßen Verfahren erhaltenen erfindungsgemäßen 5,5-Di-(methyl)-thiazolidin-4(S)-carbonsäurederivate können aus dem Reaktionsgemisch isoliert und gegebenenfalls in andere erfindungsgemäße 5,5-Di-(methyl)-thiazolidin-4(S)-carbonsäurederivate, wie Salze oder Ester, überführt werden.

Vorzugsweise wird die Umsetzung der, gegebenenfalls an ihrem Stickstoffatom substituierten, ß,ß-Di-(methyl)-cysteine der allgemeinen Formel X mit den Aldehyden der allgemeinen Formeln VI, VII, VIII, IX, IXa, XI, XIa, XII beziehungsweise XIII in Gegenwart von sauren Katalysatoren, insbesondere Salzsäure, durchgeführt.

Es ist auch bevorzugt, zur Umsetzung der, gegebenenfalls an ihrem Stickstoffatom substituierten, ß,ß-Di-(methyl)-cysteine der allgemeinen Formel X mit den Aldehyden der allgemeinen Formeln VI, VII, VIII, IX, IXa, XI, XIa, XII beziehungsweise XIII als Lösungsmittel Wasser und/oder Methanol oder Pyridin zu verwenden.

Ferner sind Gegenstand der Erfindung Arzneimittel, welche 1 oder mehr erfindungsgemäßen Verbindung(en) als Wirkstoff(e), zweckmäßig zusammen mit 1 oder mehr üblichen pharmazeutischen Konfektionierungsmittel(n), enthalten.

Die erfindungsgemäßen 5,5-Di-(methyl)-thiazolidin-4(S)-carbonsäurederivate haben nämlich überraschende überlegene pharmakologische Wirkungen, insbesondere eine Leberschutzwirkung. So regenerieren sie natürliche und experimentell hervorgerufene Leberschäden.

Diese wurden durch die folgenden Versuche nachgewiesen

Zur Untersuchung der akuten Toxizität wurden die Verbindungen in wäßriger Natriumcarbonatlösung gelöst und den Versuchstieren (CFPL-Mäuse, weiblich, 40 Tage alt) in dieser Form intraperitoneal (i.p.) oder intravenös (i.v.) verabreicht. In der folgenden Tabelle 1 sind einige charakteristische $LD_{50}$-Werte zusammengestellt.

Tabelle 1

| Verbindung Beispiel Nr. | Bezeichnung | Verabreichung | $LD_{50}$-Wert in mg/kg |
|---|---|---|---|
| 1 | 5,5-Di-/methyl/-2-/5'nitrofur-2'-yl/-thiazolidin-4(S)-carbonsäure | i. v. | 2191 |
| 14 | 5,5-Di-(methyl)-2-(hydroxymethyl)-thiazolidin-4(S)-carbonsäure | i.p. | 5000 |
| 30 | 3-/Acetyl/-5,5-di-/methyl/-2(S)-/5'-(nitro)-fur-2'-yl/-thiazolidin-4(S)-carbonsäure | i.p. | 2500 |
| 27 | 3-/Acetyl/-5,5-di-/methyl/-2(S)-/phenyl/-thiazolidin-4(S)-carbonsäure | i. p. | 1500 |
| 22 | 3-/Acetyl/-5,5-di-/methyl/-2(R)-/p-(chlor)-phenyl/-thiazolidin-4(S)-carbonsäure | i.p. | 550 |

Die Werte der akuten Toxizität sind genügend hoch, um einen therapeutischen Einsatz der Verbindungen zu ermöglichen.

Zur Untersuchung der Leberschutzwirkung wurden die Verbindungen männlichen CFY-Ratten mit einem Gewicht von 200 bis 240 g, die 18 Stunden gehungert hatten, verabreicht. Die Leberschädigung wurde experimentell mit Tetrachlorkohlenstoff, Allylalkohol beziehungsweise Galaktoseamin hervorgerufen. Untersucht wurde die Aktivität der Glutamat-Oxalacetat-Transaminase (GOT) im Serum, der Triglyceridgehalt der Leber und das histologische Bild der Leber.

Den ausgehungerten Tieren wurden die leberschädigenden Substanzen (1,5 g/kg Tetrachlorkohlenstoff peroral, 0,75 g/kg Galaktoseamin intraperitoneal und 20 mg/kg Allylalkohol intraperitoneal und 6 Stunden später die zu untersuchenden erfindungsgemäßen Verbindungen beziehungsweise als Vergleichssubstanz das bekannte Leberschutzmittel (+)-2-[3,4-Di-(hydroxy)-phenyl]-chroman-3,5,7-triol {Cianidanol} intraperitoneal in einer Dosis von 100 mg/kg verabreicht.

a) Die mit Tetrachlorkohlenstoff als leberschädigender Substanz erhaltenen Ergebnisse sind in der folgenden Tabelle 2 zusammengestellt.

Tabelle 2

| Verbindung | GOT-Aktivität in Einheiten/1 | Triglycerid in mg/mg Protein |
|---|---|---|
| $CCl_4$+5,5-Di-[methyl]-2-[5'-nitrofur-2'-yl]-thiazolidin-4(S)-carbonsäure {Beispiel 1} | 244,0±14 | 51±15 |
| $CCl_4$+5,5-Di-[methyl]-2-[fur-2'-yl]-thiazolidin-4(S)-carbonsäure {Beispiel 2} | 229,0±14 | 84±6 |
| $CCl_4$+5,5-Di-(methyl)-2-(pyrid-3'-yl)-thiazolidin-4(S)-carbonsäure {Beispiel 5} | 243,0±21 | 61±14 |
| $CCl_4$+5,5-Di-(methyl)-2-(benzofur-2'-yl)-thiazolidin-4(S)-carbonsäure {Beispiel 7} | 224,0±14 | 61±13 |

Fortsetzung der Tabelle 2

| | | |
|---|---|---|
| $CCl_4$ + 5,5-Di-(methyl)-2--(indan-5'-yl)-thiazolidin-4(S)-carbonsäure {Beispiel 8} | 234,0±23 | 76±10 |
| $CCl_4$ + 5,5-Di-[methyl]-2--[4'-(fluor)-phenyl]--thiazolidin-4(S)-carbonsäure {Beispiel 10} | 227,0±40 | 81±11 |
| $CCl_4$ + 5,5-Di-(methyl)-2-(pyrid-4'-yl)-thiazolidin--4(S)-carbonsäure {Beispiel 6} | 250,0±13 | 88±20 |
| $CCl_4$ + 5,5-Di-[methyl]-2--[(p-methylthio)-phenyl]--thiazolidin-4(S)-carbonsäure {Beispiel 11} | 235,0±19 | 70±14 |

Fortsetzung der Tabelle 2

| | | |
|---|---|---|
| $CCl_4$ + 5,5-Di-[methyl]-2-[3'-(nitro)-phenyl]-thiazolidin-4(S)-carbonsäure {Beispiel 9} | 232,0±34 | 83±19 |
| $CCl_4$ + (+)-2-[3,4-Di-(hydroxy)-phenyl]-chroman-3,5,7-triol {Cianidanol} ⟨Vergleichssubstanz⟩ | 241,0±23 | 77±7 |
| Keine [Blindversuch] | 81,7±16 | 40±8 |
| Tetrachlorkohlenstoff [$CCl_4$] | 269 ±14 | 86±17 |

In der nur mit Tetrachlorkohlenstoff behandelten Gruppe stieg durch die Schädigung der Lebergewebe die Konzentration der Glutamat-Oxalacetat-Transaminase /GOT/ im Serum auf das 3,3-fache, was darauf zurückzuführen ist, daß intrazelluläre Glutamat-Oxalacetat--Transaminase /GOT/ im Serum erscheint. Der Triglyceridgehalt der Leber stieg in dieser Gruppe auf das 2,14-fache. Diese Änderungen entsprechen dem Maß der an den histologischen Schnitten feststellbaren nekrotischen Veränderungen. Bei der Verabreichung der erfindungsgemäßen Verbindungen sank die Aktivität der Glutamat-Oxalacetat-Transaminase /GOT/ in der in der Tabelle 2 angegebenen Weise und größtenteils auch den Triglyceridgehalt der Leber.

b) Wirkung der unversuchten Verbindungen bei mit Allylalkohol hervorgerufener Vergiftung.

Nach 24 Stunden wurden die Tiere getötet und die Aktivität der Glutamat-Pyruvat-Transaminase /GPT/ im Serium wurde bestimmt. Nach der Behandlung mit Allylalkohol stieg der Serum-GPT-Spiegel von 1,81 ± 0,43 /u Mol/ml . h auf 4,16 ± 1,63 /u Mol/ml . h. Dieser Spiegel sank durch die Behandlung mit 100 mg/kg der der erfindungsgemäßen 5,5-Di-/methyl/-2-/5'-nitrofur-2'-yl/-thiazolidin-4(S)-carbonsäure {Beispiel 1} auf 2,84 ± 0,97 /u Mol/ml . h.

c) Wirkung der untersuchten Verbindungen bei mit Galaktoseamin hervorgerufener Vergiftung.

Mit Galaktoseamin können in der Leber von entzündlichen Erscheinungen umgebene Nekrosen und degenerative Gewebeverfettungserscheinungen

hervorgerufen werden. Die zu untersuchenden Verbindungen wurden den Tieren 6 Stunden nach der Galaktoseaminvergiftung intraperitoneal verabreicht. Durch die erfindungsgemäßen Verbindungen 5,5-Di-[methyl]-2-[5'-nitrofur-2'-yl]-thiazolidin-4(S)-carbonsäure [Beispiel 1] und 5,5-Di-[methyl]-2-[fur-2'-yl]-thiazolidin-4(S)-carbonsäure [Beispiel 2] gingen die Anzahl der Parenchym-Nekrosen und die Schwere der für Galaktoseaminhepatitis charakteristischen Veränderungen zurück.

Die Erfindung wird an Hand der folgenden Beispiele näher erläutert.

Die Beispiele 1 bis 22 zeigen die Umsetzung der Verbindungen der an ihrem Stickstoffatom acylsubstituierten ß,ß-Di-(methyl)-cysteine der allgemeinen Formel X mit den Aldehyden der allgemeinen Formeln VI, VII, VIII, IX, IXa, XI, XIa und XII. Als Ergebnis der Reaktion entstehen in den 2-Stellung substituierte 5,5-Di-(methyl)-thiazolidin-4(S)-carbonsäuren beziehungsweise ihre in der 3-Stellung acylierten Derivate.

Zur Umsetzung werden die ß,ß-Di-(methyl)-cysteine der allgemeinen Formel X, bei welchen R für Wasserstoff steht, in Wasser und/oder einem mit Wasser mischbaren organischen Lösungsmittel gelöst und unter Rühren beziehungsweise Schütteln werden die Aldehyde der allgemeinen Formeln VI, VII, VIII, IX, IXa, XI, XIa und XII zugegeben. Das Gemisch wird bei noch 5 Minuten bis 48 Stunden lang bei 0 bis 45 °C gerührt beziehungsweise geschüttelt, wobei das Endprodukt kristallin ausfällt. Es wird durch Filtrieren abgetrennt.

Zur Umsetzung der an ihrem Stickstoffatom acylsubstituierten ß,ß-Di-(methyl)-cysteine der allgemeinen Formel X, bei welchen R für einen gegebenenfalls wie oben angegeben substituierten, Formylbeziehungsweise Alkylcarbonylrest steht, werden diese in apolaren Lösungsmitteln, die einen sauren Katalysator enthalten, gelöst und unter Rühren beziehungsweise Schütteln werden die Aldehyde der allgemeinen Formeln VI, VII, VIII, IX, IXa XI, XIa und XII zur Lösung zugegeben. Das Gemisch wird bei Raumtemperatur 1 bis 180 Minuten lang gerührt beziehungsweise geschüttelt, wobei das Endprodukt kristallin ausfällt. Es wird durch Filtrieren abgetrennt.

Gemäß den Beispielen 23 bis 31 werden in der 2-Stellung substituierte 5,5-Di-(methyl)-thiazolidin-4(S)-carbonsäuren mit Säureanhydriden beziehungsweise Säurehalogeniden umgesetzt. Dadurch werden in der 3-Stellung acylierte Derivate von Verbindungen der Beispiele 1 bis 22 erhalten. Dabei werden die 5,5-Di-(methyl)-thiazolidin-4(S)-carbonsäurederivate der allgemeinen Formel I, bei welchen $R_3$ für Wasserstoff steht, in Wasser oder einem organischen Lösungsmittel gelöst und die Lösung wird unter ständigem Rühren mit Säureanhydriden oder Säurehalogeniden versetzt. Wenn als Reaktionsmedium Wasser verwendet wird, wird die Umsetzung bei 30 bis 100°C durchgeführt. Das Produkt fällt beim Kühlen des Reaktionsgemisches kristallin aus. Wenn ein organisches Lösungsmittel verwendet wird (Pyridin ist bevorzugt), wird als Reaktionstemperatur Raumtemperatur oder

0206197

darunter gewählt und das Endprodukt wird durch Eindampfen isoliert.

Im Beispiel 32 ist die Veresterung einer 2-substituierten 5,5-Di-(methyl)-thiazolidin-4(S)-carbonsäure mit einem Diazoalkan gezeigt. Zu diesem Zweck wird die Verbindung 5,5-Di-/methyl/-2-/5'-(nitro)-fur-2'-yl/-thiazolidin-4(S)-carbonsäure der allgemeinen Formel I, in welcher $R_2$ für Wasserstoff oder ein Alkalimetall oder Erdalkalimetall steht, in einem polaren Lösungsmittel, vorzugsweise Alkohol, gelöst und die Lösung unter Kühlen mit dem Alkylierungsmittel versetzt. Das Produkt wird durch Eindampfen gewonnen.

Die bei der Umsetzung entstehenden Isomere sind im allgemeinen dünnschichtchromatographisch nicht trennbar. Die Chromatographie an Kieselgel 60 $F_{254}$ mit einem etwas organische Säure enthaltenden Lösungsmittelgemisch als Laufmittel ergab im allgemeinen einen einzigen kompakten Fleck. - Die NMR-Spektren im Beispielteil wurden ausnahmslos in Dimethylsulfoxyd aufgenommen.

Beispiel 1

5,5-Di-[methyl]-2-[5'-nitrofur-2'-yl]-thiazolidin-4(S)-carbonsäure

15 g (0,1 Mol) D-Penicillamin (D,ß,ß-Dimethylcystein; Verbindung X mit R = H) werden unter Rühren in 190 ml ionenfreiem Wasser gelöst und die Lösung unter Rühren innerhalb von 10-12 Minuten tropfenweise mit der Lösung von 12,5 g (0,1 Mol) 5-Nitrofurfurol in 60 ml Methanol versetzt. Man rührt noch etwa eine Stunde, wobei sich ein sandfarbenes Produkt ausscheidet. Die kristalline 5,5-Di-[methyl]-2-[5'-nitrofur-2'-yl]-thiazolidin-4(S)-carbonsäure wird abfiltriert, mit 20 ml ionenfreiem Wasser gewaschen und dann an der Luft getrocknet. Man erhält 23,9 g (87,7 %) Rohprodukt. Dieses wird in 26 ml Methanol heiß gelöst, die Lösung filtriert und tropfenweise bis zum Beginn der Kristallisation mit 8-10 ml ionenfreiem Wasser versetzt. Das kristallisierende Gemisch wird in den Kühlschrank gestellt. Die Kristalle werden abfiltriert, mit 15 ml 50 %igem Methanol gewaschen und bei maximal 55 °C getrocknet. Man erhält 19,7 g (72,3 %) der Titelverbindung, die bei 147-148 °C schmilzt.

$[\alpha]_D^{20} = -159^\circ$ (c = 0,829, DMSO).

Elementaranalyse (M = 272,3)

berechnet, %: N 10,29 S 11,78

gefunden, %: N 10,14 S 11,78.

IR-Spektrum: 3304 (NH) und 1725 (CO) $cm^{-1}$ sowie 2480 $cm^{-1}$ (Hinweis auf Zwitterionenstruktur)

$^1$H-NMR: 1,34 und 1,65 (C-methyl cis), 1,40 und 1,69 (C-methyl trans), 5,77 (H-2) und 3,64 (H-4) des cis-Isomers und 5,71 (H-2) und 3,70 (H-4) ppm des trans-Isomers.

Beispiel 2

5,5-Di-[methyl]-2-[fur-2'-yl]-thiazolidin-4(S)--carbonsäure

Ein Gemisch aus 4,27 g D-Penicillamin und 2,48 ml Furfurol in 90 ml 30 %igem Methanol wird 8 Stunden lang gerührt. Anschließend wird die Lösung zur Trockne eingedampft, und das halbkristalline Produkt wird durch Verreiben mit Äther kristallisiert. Die Titelverbindung schmilzt bei 141-143 °C.

$[\alpha]_D^{20}$ = +103° (c = 0,602, DMSO).

Elementaranalyse (M = 227,3)

berechnet, %: N 6,16 S 14,10

gefunden, %: N 5,91 S 13,69.

IR: 1721 $cm^{-1}$ (CO)

$^1$H-NMR: 1,22 und 1,53 (C-Methyl, trans), 1,28 und 1,56 (C-Methyl, cis), 3,58 (H-4) und 5,70 (H-2) des cis-Isomers und 3,60 (H-4) und 5,66 (H-2) ppm des trans-Isomers.

Beispiel 3

5,5-Di-[methyl]-2-[thien-2'-yl]-thiazilidin-4(S)--carbonsäure

1,5 g (10 mMol) D-Penicillamin werden bei Raumtemperatur unter Rühren in 150 ml Methanol gelöst und zu der Lösung 0,92 ml (1,12 g; 10 mMol) Thiophen-2-(aldehyd) gegeben. Das Gemisch wird noch 48 Stunden lang gerührt und dann im Vakuum eingedampft. Der Rückstand wird unter Rühren in kochendem Benzol gelöst. Zu der Lösung wird so lange Petroläther gegeben, bis die Titelverbindung ausfällt. Ausbeute: 1,5 g (61,6 %), Schmp.: 129-131 $^{o}$C, $[\alpha]_D^{20}$ = + 56$^{o}$ (c = 0,549, DMSO).

Elementaranalyse (M = 243,3)

| | | |
|---|---|---|
| berechnet, % | N 5,75 | S 26,35 |
| gefunden, %: | N 5,31 | S 26,62 . |

$^1$H-NMR: 1,3 (s, 3H, C-$CH_3$), 1,6 (s, 3H, C-$CH_3$), 3,42 (b, NH), 3,62 (s, 1H, $\underline{H}_4$), 5,9 (s, 1H, $\underline{H}_2$), 6,98-7,52 (m, 4H, Thiophen-H).

Beispiel 4

5,5-Di-[methyl]-2-[N-(methyl)-pyrr-2'-yl]-thiazolidin-4(S)-carbonsäure

Zu der Lösung von 1,5 g (10 mMol) D-Penicillamin in 100 ml Methanol werden bei Raumtemperatur 1,09 g (1,0 ml, 10 mMol) N-[Methyl]-pyrryl-2-[aldehyd] gegeben. Nach 48 Stunden wird das Reaktionsgemisch im Vakuum eingedampft, der Rückstand wird in kochendem Benzol gelöst, die Lösung mit Aktivkohle geklärt und dann filtriert. Beim Abkühlen der Lösung scheidet sich die Titelverbindung aus. Man erhält 1,54 g (67,5 %) Produkt, das bei 126-128 °C schmilzt.

$[\alpha]_D^{20}$ = +87,5° (c = 0,471, DMSO).

Elementaranalyse (M = 240,3).

| | | |
|---|---|---|
| berechnet, %: | N 11,66 | S 13,34 |
| gefunden, %: | N 11,24 | S 13,44. |

IR: 1721 $cm^{-1}$ (CO).

$^1$H-NMR: 1,25 (s, C-$CH_3$), 1,65 (s, C-$CH_3$), 3,38 (b, NH), 3,55 (m, 3H+1H:N-$CH_3$+$\underline{H}_4$), 5,77 (s, 1H, $H_2$), 5,90 (m, 1H), 6,13 (m, 1H), 6,68 (m, 1H), Pyrrol-H.

Beispiel 5

5,5-Di-(methyl)-2-(pyrid-3'-yl)-thiazolidin-4(S)--carbonsäure

Zu der Lösung von 1,5 g (10 mMol) D-Penicillamin in 30 ml Wasser werden bei Raumtemperatur unter Rühren 0,94 ml (1,07 g, 10 mMol) Pyridin-3-(aldehyd) gegeben. Nach 10 Minuten beginnt sich das Produkt kristallin abzuscheiden. Das Gemisch wird noch eine Stunde lang gerührt und dann das Rohprodukt abgetrennt, mit Wasser gewaschen und getrocknet. Das Rohprodukt wird in kochendem Wasser gelöst, die Lösung wird filtriert und dann gekühlt, wobei das Produkt kristallin ausfällt. Ausbeute: 1,9 g (79,7 %) der Titelverbindung, die bei 164-165 °C schmilzt.

$[\alpha]_D^{20}$ = +145° (c = 0,556, DMSO).

Elementaranalyse (M = 238,30)

| | | |
|---|---|---|
| berechnet, %: | N 11,75 | S 13,46 |
| gefunden, %: | N 11,17 | S 13,69. |

IR: 1714 $cm^{-1}$ (CO).

$^1$H-NMR: 1,35 (s, C-$CH_3$), 1,6 (s, C-$CH_3$), 3,38 (b, NH), 3,38 (b, NH), 3,64 (s, $H_4$, 1H), 5,78 (s, 1H, $H_2$), 7,42 (m,/7,8/d), 8,0 (d), 8,49 (dd), 8,63 (d), Pyridyl-H.

Beispiel 6

5,5-Di-(methyl)-2-(pyrid-4'-yl)-thiazolidin-4(S)--carbonsäure

7,5 g (50 mMol) D-Penicillamin werden in 45 ml Wasser gelöst und die Lösung tropfenweise mit der Lösung von 4,7 ml (10 mMol) Pyridin-4-(aldehyd) in 21 ml Methanol versetzt. Das Gemisch wird noch 3 Stunden lang gerührt und dann der Niederschlag abfiltriert und mit Äthanol gewaschen. Die 6,5 g (54,5 %) Rohprodukt werden aus Äthanol durch Zugabe von Petroläther bei 60-80 °C umkristallisiert. Schmp.: 149-151 °C.

$[\alpha]_D^{20}$ = +42,7° (c = 1,32, DMSO).

Elementaranalyse (M = 238,3)

berechnet, %: N 11,75 S 13,46

gefunden, %: N 12,07 S 13,63 .

IR: 1722 $cm^{-1}$ (CO).

Beispiel 7

5,5-Di-(methyl)-2-(benzofur-2'-yl)-thiazolidin--4(S)-carbonsäure

1,5 g (10 mMol) D-Penicillamin werden unter Rühren in 150 ml Methanol gelöst und zu der Lösung 1,46 g (1,39 ml, 10 mMol) 2-(Formyl)-benzofuran gegeben. Nach 48

- 45 -

Stunden wird die Lösung im Vakuum eingedampft. Der sirupartige Rückstand wird in kochendem Benzol gelöst und die Lösung filtriert. Durch Zusatz von Petroläther scheidet sich die Titelverbindung kristallin aus. Ausbeute: 1,9 g (68,6 %), Schmp.: 96 °C,
$[\alpha]_D^{20}$ = +215 (c = 0,511, DMSO).
Elementaranalyse (M = 277,3)
berechnet, %: N 5,05 S 11,56
gefunden, %: N 4,90 S 11,31.
IR: 1721 $cm^{-1}$ (CO).

$^1$H-NMR: 1,33 (s, C-$CH_3$), 1,66 (s, C-$CH_3$), 3,38 (b/NH/ 3,75/s, 1H, $H_4$/5,89 /s, 1H, $H_2$), 6,92 (d/7,27/m 7,59/m), aromatischer Wasserstoff.

Beispiel 8

5,5-Di-(methyl)-2-(indan-5'-yl)-thiazolidin--4(S)-carbonsäure

1,5 g D-Penicillamin werden bei Raumtemperatur in 100 ml Methanol gelöst und zu der Lösung 1,44 g (10 mMol) 5-(Formyl)-indan gegeben. Nach 48 Stunden wird das Reaktionsgemisch eingedampft. Der Rückstand wird in Äther gelöst, die Lösung gekocht, mit Aktivkohle geklärt und dann filtriert. Das Produkt wird durch Zusatz von Petroläther ausgefällt. Das Rohprodukt wird in kochendem Äthanol gelöst, die Lösung mit Aktivkohle geklärt, filtriert und bis zum Eintreten einer Trübung tropfenweise mit Wasser versetzt. Beim Kühlen der Lösung scheidet sich die Titelverbindung kristallin aus. Man erhält 1,57 g (57,1 %) Produkt, das bei 140-142 °C schmilzt.
$[\alpha]_D^{20}$ = +18° (c = 0,551, DMSO),
Elementaranalyse (M = 275,342)
berechnet, %: S 11,65
gefunden, %: S 11,75.
IR: 1722 $cm^{-1}$ (CO).

$^1$H-NMR: 1,32 (s, C-$CH_3$), 1,6 (s, C-$CH_3$), 3,88 (b, NH), 3,62 (s, 1H, $\underline{H}_4$), 5,76 (s, 1H, $\underline{H}_2$),

Beispiel 9

5,5-Di-[methyl]-2-[3'-(nitro)-phenyl]-thiazolidin-4(S)-carbonsäure und ihr Magnesiumsalz

4,48 g (30 mMol) D-Penicillamin und 4,53 g (30 mMol) 3-(Nitro)-benzaldehyd werden in 90 ml 30 %igem Methanol gelöst. Die Lösung wird 3 Stunden lang gerührt, dann wird das ausgefallene Produkt abfiltriert und mit Methanol gewaschen. Es wird aus Methanol durch Wasserzusatz umkristallisiert. Ausbeute: 7,17 g (84,6 %).

0,22 g (5 mMol) Magnesiumoxyd werden in 30 ml Wasser suspendiert, zu der Suspension 2,82 g (10 mMol) 5,5-Di-[methyl]-2-[3'-{nitro}-phenyl]-thiazolidin-4(S)-carbonsäure gegeben und das Gemisch auf 60 °C gehalten, bis sich alles gelöst hat. Die klare Lösung wird im Vakuum zur Trockne eingedampft und der Rückstand im Exsikkator über Phosphorpentoxyd getrocknet. Die Ausbeute an dem sirupartigen Produkt ist quantitativ.

Die freie Säure schmilzt bei 139-140 °C,

$[\alpha]_D^{20}$ = -26,4° (c = 0,588, DMSO).

Elementaranalyse (M = 282,3)

berechnet, %: N 9,92 S 11,36

gefunden, %: N 9,12 S 11,02.

IR: 1726 $cm^{-1}$ (CO).

$^1H$-NMR: 1,36 (s, 3H, $CH_3$), 1,69 (s, 3H, $CH_3$), 3,57 (s, 1H, H-4), 5,76 (s, 1H, H-2), 7,65 (t, 1H), 7,95 (dd, 1H), 8,21 (dd, 1H), 8,39 (s, 1H) (arom. Signale)

Magnesiumsalz:

$[\alpha]_D^{21}$ = -45° (c = 0,63, Wasser).

Elementaranalyse (M = 586,8)

berechnet, %: N 9,55 S 10,93

gefunden, %: N 9,33 S 10,99.

Beispiel 10

5,5-Di-[methyl]-2-[4'-(fluor)-phenyl]-thiazolidin-4(S)-carbonsäure und ihr Kaliumsalz

9,0 g (60 mMol) D-Penicillamin werden unter Rühren in 200 ml Wasser gelöst und zu der Lösung 6,31 ml (60 mMol) p-(Fluor)-benzaldehyd in 40 ml Methanol gegeben. Das Gemisch wird 3 Stunden lang gerührt und der Niederschlag dann abgetrennt (13,8 g = 90 %). Das Rohprodukt wird aus Methanol durch Zusatz von Wasser umkristallisiert.

1,0 g Kaliumbicarbonat wird in 10 ml Wasser gelöst, die Lösung mit 2,55 g (10 mMol) 5,5-Di-[methyl]-2-[4'-(fluor-)-phenyl]-thiazolidin-4(S)-carbonsäure versetzt und auf dem Dampfbad auf 60 °C erwärmt. Unter Kohlendioxydentwicklung geht die Substanz in Lösung. Die Lösung wird bis zur sirupartigen Konsistenz eingedampft und dann im Exsikkator über Phosphorpentoxyd bis zur Gewichtskonstanz getrocknet. Das Salz ist ein hygroskopisches Pulver.

Die freie Säure schmilzt bei 126-127 °C,

$[\alpha]_D^{25}$ = +86,9° (c = 0,473, DMSO).

Elementaranalyse (M = 255,3)

berechnet, %: N 5,49 S 12,56

gefunden, %: N 5,56 S 12,47.

Beispiel 11

5,5-Di-[methyl]-2-[(p-methylthio)-phenyl]-thiazolidin-4(S)-carbonsäure

4,48 g (30 mMol) D-Penicillamin und 3,99 ml (30 mMol) p-(Methylthio)-benzaldehyd werden in 90 ml 30 %igem wäßrigem Methanol 6 Stunden lang gerührt. Der weiße Niederschlag wird abfiltriert. Ausbeute: 8,26 g (97,1 %). Die Substanz kristallisiert aus Methanol bei Zugabe von wenig Wasser. Schmp.: 150-152 °C,

$[\alpha]_D^{20}$ = +174,4° (c = 0,516, DMSO).

0206197

Elementaranalyse (M = 283,4)
berechnet, %: N 4,94 S 22,63
gefunden, %: N 4,85 S 22,31.
IR: 2960 und 2914 $cm^{-1}$ ($CH_3$), 1720 $cm^{-1}$ (CO).

$^1$H-NMR: 1,30 (s, $C\text{-}CH_3$), 1,36 (s, $C\text{-}CH_3$), 1,55 (s, $C\text{-}CH_3$), 1,65 (s, $C\text{-}CH_3$), 2,45 (s, 4'-$CH_3$), 2,48 (s, 4'-$CH_3$), 3,56 (s, H-4), 3,69 (s, H-4), 5,60 (s, H-2), 5,82 (s, H-2), 7,17-7,45 (m, arom.).

Beispiel 12

5,5-Di-[methyl]-2-[2'-(carboxy)-phenyl]-thiazolidin--4(S)-carbonsäure

6 g (30 mMol) 2-(Carboxy)-benzaldehyd und 6 g (30 mMol) D-Penicillamin werden in 44 ml Wasser 3 Stunden lang gerührt. Der Niederschlag wird abfiltriert und mit wäßrigem Methanol gewaschen. Ausbeute: 9,86 g (82,6 %). Das Rohprodukt wird aus wäßrigem Methanol umkristallisiert.
Schmp.: 185-186 °C, $[\alpha]_D^{18}$ = +385° (c = 0,51, Methanol)
Elementaranalyse (M = 281,3)
berechnet, %: N 4,98 S 11,40
gefunden, %: N S 11,39.

IR: 1763 und 1705 $cm^{-1}$ (CO).

$^1$H-NMR: 1,50 (s, 3H, $CH_3$), 1,60 (s, 3H, $CH_3$), 3,41 (b, s, 2H, OH), 4,51 (s, 1H, H-4), 6,45 (s, 1H, H-2), 7,87 (m, 4H, arom.)

Beispiel 13

5,5-Di-[methyl]-2-[3',4'-di-(methoxy)-2'-(carboxy)--phenyl]-thiazolidin-4(S)-carbonsäure

2,1 g (10 mMol) Opiansäure werden in 30 ml kochendem Wasser gelöst. Die Lösung wird auf etwa 60 °C gekühlt und dann mit 1,5 g (10 mMol) D-Penicillamin versetzt. Aus der opalisierenden Lösung scheidet sich ein öliges Produkt ab, das über Nacht kristallisiert. Ausbeute: 2,75 g

(80,5 %). Das Produkt wird aus Methanol durch Zusatz von Wasser umkristallisiert und schmilzt dann bei 90-91 °C.
$[\alpha]_D$ = 326° (c = 0,772, Methanol).
Elementaranalyse (M = 341,4)
berechnet, %: N 4,10 S 9,39
gefunden, %: N 4,21 S 9,21.
IR: 1721 und 1690 $cm^{-1}$ (CO).

$^1$H-NMR: 1,50 (s, 3H, $CH_3$), 1,60 (s, 3H, $CH_3$), 3,50 (br, OH), 3,79 (s, 3H, $OCH_3$), 3,84 (s, 3H, $OCH_3$), 4,43 (s, 1H, H-4), 6,35 (s, 1H, H-2), 7,35 (q, 2H, arom.)

Beispiel 14

5,5-Di-(methyl)-2-(hydroxymethyl)-thiazolidin--4(S)-carbonsäure

3,0 g (50 mMol) Glykolaldehyd und 7,5 g (30 mMol) D-Penicillamin werden in 30 ml 30 %igem Methanol 6 Stunden lang gerührt. Der Niederschlag wird abfiltriert und aus 40 ml kochendem Wasser umkristallisiert. Man erhält 2,17 g (22,7 %) der Titelverbindung, die bei 179-180 °C schmilzt.
$[\alpha]_D^{24}$ = +167° (c = 0,62, 10 %ige $NaHCO_3$-Lösung)
Elementaranalyse (M = 191,2)
berechnet, %: N 7,32 S 16,76
gefunden, %: N 7,44 S 16,78.
IR: 1635 $cm^{-1}$ (CO).
$^1$H-NMR: 1,20 und 1,55 (C-Methyl, cis), 3,20 ($CH_2$-A), 3,41 ($CH_2$-B), 3,44 (H-4) und 4,58 (H-2), beide cis.

Beispiel 15

5,5-Di-[methyl]-2-[3',5'-di-(brom)-4'-(hydroxy)--phenyl]-thiazolidin-4(S)-carbonsäure

5,6 g (20 mMol) 3,5-Di-(brom)-4-(hydroxy)-benzaldehyd und 3 g (20 mMol) D-Penicillamin werden in 100 ml 30 %igem Methanol 16 Stunden lang gerührt. Der Niederschlag wird abfiltriert und mit wäßrigem Methanol gewaschen. Ausbeute: 6,77 g (82,3 %). Das Produkt wird aus einem Gemisch von Äthanol, Dimethylformamid und Wasser kristallisiert und schmilzt dann bei 154-155 °C.

$[\alpha]_D^{25}$ = +87° (c = 1,362, DMSO).
Elementaranalyse (M = 411,1)
berechnet, %: N 3,41 S 7,80 Br 38,88
gefunden, %: N 3,56 S 7,77 Br 38,22 .
IR: 1708 $cm^{-1}$ (CO).

$^1H$-NMR: 1,27 (s, $CH_3$), 1,30 (s, $CH_3$), 1,52 (s, $CH_3$), 1,61 (s, $CH_3$), 3,50 (s, H-4), 3,57 (s, H-4), 5,50 (s, H-2), 5,74 (s, H-2), 3,45 (br, OH), 7,46, 7,70 (arom.), 9,98 (Br, OH).

Beispiel 16

5,5-Di-[methyl]-2-[2'-(methyl)-5'-(hydroxymethyl)-3'-(hydroxy)-pyrid-4'-yl]-thiazolidin-4(S)-carbonsäure

1,5 g (10 mMol) D-Penicillamin und 0,56 g Kaliumhydroxyd werden in 10 ml Wasser gelöst. Die Lösung wird mit 2,03 g (10 mMol) Pyridoxal-hydrochlorid versetzt und noch 16 Stunden lang gerührt. Dann wird der Niederschlag abfiltriert. Ausbeute: 2,26 g (75,7 %). Das Produkt wird durch Auskochen in Äthanol gereinigt. Schmp.: 201-202 °C, $[\alpha]_D^{25}$ = +68,1°.
Elementaranalyse (M = 298,3)
berechnet, %: N 9,39 S 10,74
gefunden, %: N 9,30 S 10,54 .
IR: 3340 und 3292 $cm^{-1}$ (OH), 1725 $cm^{-1}$ (CO).
$^1H$-NMR: 1,66 und 1,83 (C-Methyl, trans), 1,71 und 1,91 (C-Methyl, cis), 2,83 (arom. Methyl), ca. 5 ($CH_2$), 6,56 (H-2) cis, 6,68 (H-4) trans.

Beispiel 17

5,5-Di-[methyl]-2-(2',4'-di-(chlor)-phenyl]-thiazolidin-4(S)-carbonsäure

3,0 g (20 mMol) D-Penicillamin werden in 65 ml Wasser gelöst. Zu der Lösung gibt man die Lösung von 3,5 g (20 mMol) 2,4-Di-(chlor)-benzaldehyd in 17 ml Methanol. Das Gemisch wird 16 Stunden lang gerührt und der Niederschlag

dann abfiltriert und mit 50 %igem Methanol gewaschen.
Ausbeute: 5,82 g (95 %), Schmp.: 150-151 °C,
$[\alpha]_D^{20}$ = 353° (c = 0,45, DMSO).
Elementaranalyse (M = 306,2)
berechnet, %: Cl 23,16 S 10,47
gefunden, %: Cl 23,79 S 10,60.
IR: 1700 $cm^{-1}$ (CO).

$^1$H-NMR: 1,30 und 1,50 (C-Methyl, cis), 3,35 (NH), 3,71 (H-4, cis), 5,88 (H-2, cis).

Beispiel 18

5,5-Di-(methyl)-2-(p-tolyl)-thiazolidin-4(S)-carbonsäure

1,50 g (10 mMol) D-Penicillamin und 1,18 ml (10 mMol) p-Toluylaldehyd werden in 30 ml 30 %igem Methanol eine Stunde lang gerührt. Die ausgefallenen Kristalle werden abfiltriert und mit 30 %igem Methanol gewaschen. Ausbeute: 2,25 g (89,5 %). Das Produkt kann aus Methanol durch Zusatz von Wasser umkristallisiert werden. Schmp.: 89-90 °C,
$[\alpha]_D$ = 53,5° (c = 0,784, DMSO).
Elementaranalyse (M = 251,3)
berechnet, %: N 5,57 S 12,76
gefunden, %: N 5,51 S 12,11.
IR: 1740 $cm^{-1}$ (CO).

$^1$H-NMR: 1,26 und 1,54 (C-Methyl, cis), 1,36 und 1,69 (C-Methyl, trans), 2,28 (arom. $CH_3$), 3,58 (H-4, cis), 5,81 (H-2, cis), 3,64 (H-4, trans), 5,58 (H-2, trans).

Beispiel 19

5,5-Di-[methyl]-2-[4'-(hydroxy)-phenyl]-thiazolidin-4(S)-carbonsäure

1,49 g (10 mMol) D-Penicillamin werden in 30 ml 30 %igem Methanol gelöst und 1,26 g (10 mMol) p-(Hydroxy)-benzaldehyd in 20 ml 30 %igem Methanol zugegeben. Das Gemisch wird 20 Stunden lang gerührt, dann wird der Niederschlag abfiltriert und aus wäßrigem Äthanol umkristallisiert. Ausbeute: 2,4 g (94,8 %) der Titelverbindung, die bei 207-209 °C schmilzt.

$[\alpha]_D$ = +91,3° (c = 0,2, Dimethylformamid)
Elementaranalyse (M = 253,2)
berechnet, %: N 5,53 S 12,64
gefunden, %: N 5,43 S 12,60.
IR: 1630, 1608, 1589 $cm^{-1}$ (CO).

$^1$H-NMR: 1,32 und 1,57 (C-Methyl, cis), 1,34 und 1,66 (C-Methyl, trans), 2,71 (OH, cis), 2,88 (OH, trans), 3,56 (H-4) und 5,76 (H-2), beide cis, 3,62 (H-4) und 5,55 (H-2), beide trans.

Beispiel 20

5,5-Di-[methyl]-2-[2'-(hydroxy)-phenyl]-thiazolidin-4(S)-carbonsäure

4,5 g (30 mMol) D-Penicillamin und 3,16 ml (30 mMol) Salicylaldehyd werden in 90 ml 30 %igem Methanol 3 Stunden lang gerührt. Der Niederschlag wird abfiltriert, mit 50 %igem Methanol gewaschen und dann getrocknet. Man erhält 7,05 g (92,7 %) der Titelverbindung, die aus Methanol kristallisiert wird und dann bei 179-181 °C schmilzt.
$[\alpha]_D$ = 129,8° (c = 0,48, DMF).
Elementaranalyse (M = 253,2)
berechnet, %: N 5,53 S 12,64
gefunden, %: N 5,36 S 12,25.
IR: 1638 $cm^{-1}$.

$^1$H-NMR: 1,30 und 1,48 (C-Methyl, cis), 1,36 und 1,69 (C-Methyl, trans), 3,16 (OH), 3,64 (H-4) und 5,76 (H-2), beide trans, und 3,67 (H-4) sowie 5,90 (H-2), beide cis.

Beispiel 21

3-[Acetyl]-5,5-di-[methyl]-2(R)-[p-(fluor)-phenyl]-thiazolidin-4(S)-carbonsäure

5,74 g (30 mMol) N-[Acetyl]-D-penicillamin und 3,2 ml p-(Fluor)-benzaldehyd werden in 20 ml mit Chlorwasserstoffgas gesättigtem Äther gerührt. Nach 5-6 Minuten entsteht ein

Öl, das nach 30 Minuten bei Anreiben kristallisiert. Der Niederschlag wird abfiltriert und mit Äther gewaschen. Ausbeute: 7,81 g (87,5 %). Das Produkt wird aus Äthylacetat umkristallisiert und schmilzt dann bei 144-146 °C.
$[\alpha]_D^{22} = +206,9°$.
Elementaranalyse (M = 297,3)
berechnet, %: N 4,71 S 10,78
gefunden, %: N 4,80 S 11,12.
IR: 1742 $cm^{-1}$ (CO).

$^1$H-NMR: 1,60 (s, 3H, $CH_3$), 1,45 (s, 3H, $CH_3$), 1,83 (s, 3H, N-$COCH_3$), 4,71 (s, 1H, H-4), 6,32 (s, 1H, H-2), 7,05-7,35 (Multiplett, 4H, arom.).

Beispiel 22

3-[Acetyl]-5,5-di-[methyl]-2(R)-[p-(chlor)-phenyl]-thiazolidin-4(S)-carbonsäure

5,73 g (30 mMol) N-[Acetyl]-D-penicillamin werden in 18 ml ätherischer Salzsäure suspendiert und zu der Suspension unter Rühren 4,2 g (30 mMol) p-(Chlor)-benzaldehyd gegeben. Nachdem sich alles gelöst hat (etwa 12 Minuten), fällt das Produkt aus. Es wird mit Äther gewaschen. Ausbeute: 8,32 g (88,4 %). Nach Umkristallisieren aus Äthylacetat schmilzt das Produkt bei 197-198 °C.
$[\alpha]_D = 230,5°$ (c = 0,59, Methanol).
Elementaranalyse (M = 313,8)
berechnet, %: N 4,46 S 10,22 Cl 11,30
gefunden, %: N 4,41 S 10,18 Cl 11,11.
IR: 1742 $cm^{-1}$ (CO).

$^1$H-NMR: 1,45 (s, 3H, $CH_3$), 1,59 (s, 3H, $CH_3$), 1,85 (s, 3H, $NHCOCH_3$), 4,71 (s, 1H, 4-H), 6,32 (s, 1H, 2H), 7,33 (Multiplett, 4H, arom.)

Beispiel 23

3-[Acetyl]-5,5-di-[methyl]-2(S)-[p-(chlor)-phenyl]--thiazolidin-4(S)-carbonsäure

2,71 g (10 mMol) 5,5-Di-[methyl]-2(S)-[p-(chlor)-phenyl]-thiazolidin-4(S)-carbonsäure werden in 5,6 ml kochendem Wasser suspendiert und 5,6 ml Essigsäureanhydrid zugesetzt. Man kocht das Gemisch 8 Minuten lang, wobei sich alles löst. Beim Abkühlen beginnt sich das Produkt abzuscheiden. Ausbeute: 2,39 g (76,1 %). Das Produkt wird aus Wasser durch Zusatz von Äthanol umkristallisiert und schmilzt dann bei 203-205 °C. $[\alpha]_D^{22}$ = 111,5° (c = 1,44, DMSO)

Elementaranalyse (M = 319,8)

berechnet, %: Cl 11,30 S 10,22

gefunden, %: Cl 11,94 S 9,97.

IR: 1730 $cm^{-1}$ (CO) und 1626 $cm^{-1}$ (Amid).

$^1$H-NMR: 1,36 (s, 3H, $CH_3$), 1,61 (s, 3H, $CH_3$), 1,86 (N-$COCH_3$), 4,60 (s, 1H, H-4), 6,27 (s, 1H, H-2), 7,33 (s, 2H, H-3',5'), 7,76 (s, 2H, H-2',6').

Die Ausgangsverbindung wurde analog Beispiel 17, jedoch ausgehend von p-Chlorbenzaldehyd hergestellt.

Beispiel 24

3-[Acetyl]-5,5-di-[methyl]-2(S)-[2'-(acetoxy)-phenyl]--thiazolidin-4(S)-carbonsäure

Ein Gemisch aus 1,0 g 5,5-Di-[methyl]-2-[2'-(hydroxy)-phenyl]--thiazolidin-4(S)-carbonsäure, 7 ml Pyridin und 3 ml Essigsäureanhydrid wird 20 Stunden lang stehengelassen. Dann wird das Reaktionsgemisch eingedampft, und 3x20 ml Toluol werden von ihm abdestilliert. Das kristalline Rohprodukt wird aus einem Gemisch von Aceton und Petroläther umkristallisiert. Man erhält 0,9 g (67,5 %) der Titelverbindung, die bei 214-216 °C schmilzt.

$[\alpha]_D^{22}$ = +34° (c = 0,47, Chloroform).

0206197

Elementaranalyse (M = 337,18)
berechnet, %: N 4,13 S 9,40
gefunden, %: N 4,00 S 9,33
IR: 1778 und 1760 $cm^{-1}$ (CO) und 1614 $cm^{-1}$ (Amid).
$^1H$-NMR: 1,28 und 1,58 (C-Methyl), 1,73 (N-COC$\underline{H}_3$), 2,29 (O-COC$\underline{H}_3$), 3,36 (NH), 4,47 (H-4), 6,34 (H-2).

Die Ausgangsverbindung wurde gemäß Beispiel 20 hergestellt.

**Beispiel 25**

3-[Acetyl]-5,5-di-[methyl]-2-(S)-[2',4'-di-(chlor)-phenyl]-thiazolidin-4(S)-carbonsäure

5,5-Di-[methyl]-2-[2',4'-di-(chlor)-phenyl]-thiazolidin-4(S)-carbonsäure wird in 5,56 ml Wasser kochend suspendiert. Das Gemisch wird mit 5,56 ml Essigsäureanhydrid versetzt, etwa 6 Minuten lang auf 100 °C gehalten, dann abgekühlt und mit etwa 10 ml Wasser verdünnt. Die Kristalle werden abfiltriert. Ausbeute: 2,57 g (73,8 %). Das Produkt wird aus wäßrigem Methanol umkristallisiert.
Schmelzpunkt: 233-235 °C.
$[\alpha]_D^{22} = -142°$.
Elementaranalyse (M = 350,08)
Berechnet, %: S 9,20
gefunden, %: S 9,08.
IR: 1738 (CO) $cm^{-1}$.

$^1H$-NMR: 1,27 (s, $CH_3$), 1,37 (s, $CH_3$), 1,56 (s, $CH_3$), 1,60 (s, $CH_3$), 2,02 (N-$COCH_3$), 4,49 (s, H-4), 4,78 (s, H-4), 6,27 (s, H-2), 6,44 (s, H-2), 7,60-8,00 (Multiplett, 3H, arom.).

Die Ausgangsverbindung wurde gemäß Beispiel 17 hergestellt.

Beispiel 26

3-[Acetyl]-5,5-di-[methyl]-2(S)-[p-tolyl]-thiazolidin-4(S)-carbonsäure

Zu einer Suspension von 2,51 g (10 mMol) 5,5-Di-[methyl]-2-[p-tolyl]-thiazolidin-4(S)-carbonsäure in 5,6 ml kochendem Wasser werden 5,6 ml Essigsäureanhydrid gegeben. Das Gemisch wird 5 Minuten lang auf 100 °C gehalten. Nach dem Abkühlen fallen Kristalle aus, die aus Isopropanol umkristallisiert werden. Ausbeute: 2,15 g (73,3 %).

Schmp.: 214-218 °C, $[\alpha]_D^{19}$ = -101° (c = 0,71, Methanol)

Elementaranalyse (M = 293,3)

berechnet, %: N 4,77 S 10,93

gefunden, %: N 4,66 S 10,91.

IR: 1732 $cm^{-1}$ (CO).

$^1$H-NMR: 1,35 (s, 3H, $CH_3$), 1,59 (s, 3H, $CH_3$), 1,81 (s, 3H, N-$COCH_3$), 2,30 (s, 3H, arom.-$CH_3$), 4,58 (s, 1H, H-4), 6,23 (s, 1H, H-2), 7,14-7,63 (Multiplett, 4H, arom.)

Die Ausgangsverbindung wurde gemäß Beispiel 18 hergestellt.

Beispiel 27

3-[Acetyl]-5,5-di-[methyl]-2(S)-[phenyl]-thiazolidin-4(S)-carbonsäure

0,47 g (2 mMol) 5,5-Di-[methyl]-2(S)-[phenyl]-thiazolidin-4(S)-carbonsäure werden in 5 ml Pyridin suspendiert und mit Eiswasser auf 5 °C gekühlt. Dann werden 0,17 g Acetylchlorid zugetropft. Das Gemisch wird noch eine Stunde lang gerührt und dann in 60 ml 2n eiskalte Schwefelsäure eingegossen. Nach einigen Stunden wird der Niederschlag abfiltriert, mit Wasser gewaschen und dann getrocknet. Das Produkt wird durch Zusatz von Wasser aus Äthanol umkristallisiert. Man erhält 0,38 g (70,3 %) der Titelverbindung, die bei 216-217 °C schmilzt.

$[\alpha]_D^{20}$ = -109° (c = 0,686, Methanol)
Elementaranalyse (M = 270,3)
berechnet, %: N 5,18 S 11,48
gefunden, %: N 5,30 S 11,27
IR: 1732 $cm^{-1}$ (CO), 1621 $cm^{-1}$ (Amid)

$^1$H-NMR: 1,37 (s, 3H, $CH_3$), 1,62 (s, 3H, $CH_3$), 1,85 (s, 3H, N-$COCH_3$), 4,60 (s, 1H, H-4), 6,24 (s, 1H, H-2), 7,30-7,75 (Multiplett, 5H, arom.)

Die Ausgangsverbindung wurde analog Beispiel 18, jedoch ausgehend von Benzaldehyd hergestellt.

## Beispiel 28

3-[Acetyl]-5,5-di-[methyl]-2(S)-[4'-(acetoxy)-phenyl]-thiazolidin-4(S)-carbonsäure

1,5 g 5,5-Di-[methyl]-2-[p-(hydroxy)-phenyl]-thiazolidin-4(S)-carbonsäure werden in einem Gemisch aus 15 ml Pyridin und 3 ml Essigsäureanhydrid 20 Stunden lang stehen gelassen. Dann wird das Gemisch eingedampft. Vom Rückstand werden 2x30 ml Toluol abdestilliert. Der Rückstand wird aus einem Gemisch von Aceton und Petroläther umkristallisiert.

Man erhält 1,5 g (75 %) der Titelverbindung, die bei 199-201 °C schmilzt.
$[\alpha]_D^{22}$ = -61,7° (c = 0,52, Chloroform)
Elementaranalyse (M = 337,18)
berechnet, %: N 4,12 S 9,43
gefunden, %: N 4,15 S 9,42
IR: 1734-1770 $cm^{-1}$ (CO)

$^1$H-NMR: 1,30 und 1,51 (C-Methyl), 2,27 (N-$COCH_3$), 3,36 (OH), 4,55 (H-4), 6,45 (H-2).

Die Ausgangsverbindung wurde gemäß Beispiel 19 hergestellt.

Beispiel 29

3-[Acetyl]-5,5-di-[methyl]-2(S)-[p-(fluor)-phenyl]-
-thiazolidin-4(S)-carbonsäure

5,1 g (20 mMol) 5,5-Di-[methyl]-2-[p-(fluor)-phenyl]-thiazolidin-4(S)-carbonsäure werden in 11,2 ml kochendem Wasser suspendiert und zu der Suspension tropfenweise 11,2 ml Essigsäureanhydrid gegeben. Man hält das Gemisch 5 Minuten lang auf 100 °C. Nach dem Abkühlen fallen Kristalle aus. Man erhält 5,83 g (98 %) Produkt, das aus einem Äthanol-Äther-Gemisch umkristallisiert wird.

Schmp.: 193,5 °C, $[\alpha]_D^{22}$ = -96,8° (c = 0,62, Methanol)

Elementaranalyse (M = 297,3)

berechnet, %: N 4,71 S 10,78

gefunden, %: N 4,63 S 10,85

IR: 1736 $cm^{-1}$ (CO), 1626 $cm^{-1}$ (Amid)

$^1$H-NMR: 1,37 (C-$CH_3$, s, 3H), 1,62 (C-$CH_3$, s, 3H), 1,86 (N-$COCH_3$), 4,60 (H-4, s, 1H), 6,24 (H-2, s, 1H), 7,05 (H'-3, H'-5, s, 2H), 7,80 (H'-2, H'-6, s, 2H).

Die Ausgangsverbindung wurde gemäß Beispiel 10 hergestellt.

Beispiel 30

3-[Acetyl]-5,5-di-[methyl]-2(S)-[5'-(nitro)-fur-2'-yl]-
-thiazolidin-4(S)-carbonsäure

2,72 g (10 mMol) 5,5-Di-[methyl]-2-[5'-(nitro)-fur-2'-yl]-thiazolidin-4(S)-carbonsäure werden in 5,6 ml Wasser gelöst. Die Lösung wird auf dem Dampfbad auf 100 °C erwärmt, mit 5,6 ml Essigsäureanhydrid versetzt und weitere 3 Minuten erwärmt. Nach Zusatz von Wasser scheidet sich das Produkt (2,6 g, 84,3%) aus. Schmelzpunkt des Endproduktes: 191-194 °C, $[\alpha]_D^{25}$ = -315,8° (c = 0,938, DMSO)

Elementaranalyse (M = 308,3)

berechnet, %: N 9,08 S 10,40

gefunden, %: N 8,72 S 10,13

IR: 1740 $cm^{-1}$ (CO)

$^1H$-NMR: 1,30 und 1,41 ($CH_3$), 1,57 und 1,61 ($CH_3$), 2,03 ($N-COCH_3$), 3,45 (OH), 4,50 und 4,79 (H-4), 6,37 und 6,71 (H-2), 6,95, 7,16, 7,71 und 7,76 (arom.)

Die Ausgangsverbindung wurde gemäß Beispiel 1 hergestellt.

Beispiel 31

3-[2'-{p-(Chlorphenoxy)}-isobutyryl]-5,5-di-[methyl]-2(S)--[phenyl]-thiazolidin-4(S)-carbonsäure

Eine Lösung von 1,17 g (5 mMol) 5,5-Di-[methyl]-2-[phenyl]--thiazolidin-4(S)-carbonsäure und 0,7 ml Triäthylamin in 20 ml Dichlormethan wird auf 0 °C gekühlt und unter Rühren mit der Lösung von 2-(p-Chlorphenoxy)-isobuttersäurechlorid in 4 ml Dichlormethan versetzt. Das Gemisch wird 3 Stunden lang gerührt und dann mit Wasser ausgeschüttelt. Die organische Phase wird über Magnesiumsulfat getrocknet und dann eingedampft. Das sirupartige Produkt wird aus Methanol durch Zusatz von Wasser kristallisiert. Man erhält 1,39 g (64 %) der Titelverbindung, die bei 177-178 °C schmilzt.

$[\alpha]_D^{21} = 0$ (c = 0,94, DMSO).

Elementaranalyse (M = 433,9)

berechnet, %: N 3,23 S 7,39 Cl 8,17

gefunden, %: N 3,11 S 7,68 Cl 8,14

IR: 3017 und 2975 ($CH_3$), 1740 (CO), 1609 (Amid II) $cm^{-1}$.

$^1H$-NMR: 1,32, 1,39, 1,44 und 1,55 (4x3s, $CH_3$), 5,04 (s, 1H, H-4), 6,26 (s, 1H, H-2), 6,76-7,36 (Multiplett, 9H, arom.)

Die Ausgangsverbindung wurde analog Beispiel 18, jedoch ausgehend von Benzaldehyd hergestellt.

0206197

Beispiel 32

5,5-Di-[methyl]-2(S)-[5'-(nitro)-fur-2'-yl]-thiazolidin--4(S)-carbonsäuremethylester

2,72 g (10 mMol) wie im Beispiel 1 beschrieben hergestellte 5,5-Di-[methyl]-2-[5'-(nitro)-fur-2'-yl]-thiazolidin-4(S)-carbonsäure werden bei Raumtemperatur in 50 ml Methanol gelöst und zu der Lösung unter Kühlen eine aus 4,45 g (43,2 mMol) Nitrosomethylharnstoff bereitete ätherische Diazomethanlösung gegeben. Das Reagens wird in kleinen Dosen so lange zugesetzt, bis die gelbe Farbe bleibt. Nach etwa einer Stunde wird der Überschuß des Diazomethans durch ein paar Tropfen Eisessig zersetzt. Durch Verdampfen des Lösungsmittels erhält man das Produkt in Form eines blaßgelben Sirups.

Massenspektrum: 286 ($M^+$) ($M$ = 286,304)

IR: 1743 $cm^{-1}$ (CO) (in KBr)

$[\alpha]_D^{24}$ = 194° (c = 0,670, Chloroform)

$^1$H-NMR: 1,3 (3H, C-$CH_3$), 1,68 (3H, C-$CH_3$), 3,8 (4H, $H_4$+ O-$CH_3$), 5,7 (1H, $H_2$), 6,63/1H, 7,26/1H $H_3$ und $H_4$

Beispiel 33

Herstellung von Dragees mit einem erfindungsgemäßen Wirkstoff

| | |
|---|---|
| 5,5-Di-[methyl]-2-[5'-nitrofur-2'-yl]-thiazolidin-4(S)-carbonsäure | 100 mg |
| Kartoffelstärke | 30 mg |
| Milchzucker | 50 mg |
| weiße Gelatine | 10 mg |
| Carboxymethyl-cellulose-Na | 1 mg |
| Polivdon | 2 mg |
| Talk | 36 mg |
| Magnesiumstearat | 4 mg |
| kolloidales $SiO_2$ | 3 mg |
| Titandioxyd | 1 mg |
| Eisenoxydpigment | 2 mg |
| Saccharose | 106 mg |
| Calciumcarbonat | 55 mg |

- 61 -

Der Wirkstoff wird mit den den Kern der Tablette bildenden Hilfsstoffen homogenisiert, granuliert, getrocknet und das Gemisch in an sich bekannter Weise zu Tabletten gepreßt. Diese werden im Suspensionsverfahren mit einer Zuckerschicht überzogen.

Beispiel 34

Herstellung von Dragees mit einem erfindungsgemäßen Wirkstoff.

| | | |
|---|---|---|
| 5,5-Di-[methyl]-2-[fur-2'-yl]-thiazolidin-4(S)-carbonsäure | 100 | mg |
| Milchzucker | 21,6 | mg |
| Carboxymethylcellulose-Na | 15 | mg |
| mikrokristalline Cellulose | 100 | mg |
| Talk | 11 | mg |
| Magnesiumstearat | 4 | mg |
| kolloidales Siliciumdioxyd | 2 | mg |
| Methacrylsäureacrylester-Polymer | 0,3 | mg |
| Titandioxyd | 3 | mg |
| Eisenoxydpigment | 0,1 | mg . |

Der Wirkstoff wird mit den Hilfsstoffen homogenisiert. Aus dem Gemisch werden in an sich bekannter Weise Tabletten von 200 mg Gewicht hergestellt. Diese werden mit einem Filmüberzug versehen.

Patentansprüche

0206197

## Patentansprüche

1.) 5,5-Di-(methyl)-thiazolidin-4(S)-carbonsäurederivate der allgemeinen Formel

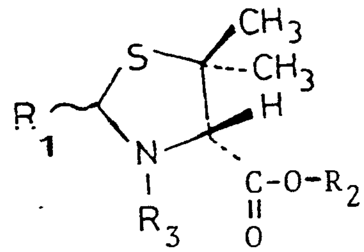

I ,

worin

$R_1$ für einen, gegebenenfalls substituierten, Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest oder einen, gegebenenfalls durch eine Carboxy- oder Hydroxylgruppe substituierten, Alkylrest mit 1 bis 4 Kohlenstoffatom(en) oder Alkenylrest mit 2 bis 4 Kohlenstoffatomen steht,

$R_2$ Wasserstoff, ein Alkalimetall- oder Erdalkalimetallatom, einen, gegebenenfalls substituierten, Alkylrest mit 1 bis 4 Kohlenstoffatom(en) oder einen, gegebenenfalls substituierten, Arylrest bedeutet und

$R_3$ Wasserstoff, einen gegebenenfalls substituierten, Alkyl- oder Acylrest mit 1 bis 4 Kohlenstoffatom(en) oder einen, gegebenenfalls substituierten, Arylrest darstellt

sowie ihre Salze.

2.) 5,5-Di-(methyl)-thiazolidin-4(S)-carbonsäurederivate der allgemeinen Formel I, dadurch gekennzeichnet, daß

$R_1$ für einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstofatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n] Alkylcarbonylrest(e) mit 2 bis 4 Kohlenstoffatomen substituierten, Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest oder einen, gegebenenfalls

durch eine Carboxyl- oder Hydroxygruppe substituierten, Alkylrest mit 1 bis 4 Kohlenstoffatom(en) oder Alkenylrest mit 2 bis 4 Kohlenstoffatomen steht,

$R_2$ Wasserstoff, ein Alkalimetall oder Erdalkalimetall, einen, gegebenenfalls durch 1 oder mehr Nitrogruppe(n), Aminogruppe(n), Halogenatome(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen substituierten, Alkylrest mit 1 bis 4 Kohlenstoffatom(en) oder, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit

2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen substituierten, Arylrest bedeutet und

$R_3$ Wasserstoff einen, gegebenenfalls durch 1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen substituierten, Alkylrest mit 1 bis 4 Kohlenstoffatom(en), einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Alkylthiorest(e) mit

1 bis 4 Kohlenstoffatom(en) und/oder, gegebenenfalls durch 1 oder mehr Halogenatom(e) substituierte[n], Phenoxyrest(e) substituierten, Formyl- beziehungsweise Alkylcarbonylrest mit 2 bis 4 Kohlenstoffatomen oder einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen substituierten, Arylrest darstellt,

sowie ihre Salze.

3.) 5,5-Di-(methyl)-thiazolidin-4(S)-carbonsäurederivate nach Anspruch 1 oder 2, dadurch gekennzeichnet, daß der Alkylrest, für den $R_1$ stehen kann, oder der beziehungsweise die Alkylrest(e), Hydroxyalkylrest(e), Alkoxyrest(e) und/oder Alkylthiorest(e), durch welche[n] der Furyl-, Thienyl-, Pyrryl-, Benzofuryl-, Benzothienyl-, Benzopyrryl-,

Phenyl-, Pyridyl-, Chinolinyl-, Isochinolinyl- oder Indanylrest, für den $R_1$ stehen kann, substituiert sein kann, und/oder der beziehungsweise die Hydroxyalkylrest(e), Alkoxyrest(e) und/oder Alkylthiorest(e), durch welche[n] der Alkyl- oder Arylrest, für den $R_2$ stehen kann, substituiert sein kann, oder der beziehungsweise die Alkylrest(e), durch welche[n] der Arylrest, für den $R_2$ stehen kann, substituiert sein kann, und/oder der beziehungsweise die Hydroxyalkylrest(e), Alkoxyrest(e) und/oder Alkylthiorest(e), durch welche[n] der Alkylrest oder Formyl- beziehungsweise Alkylcarbonylrest, für den $R_3$ stehen kann, substituiert sein kann, oder der beziehungsweise die Alkylrest(e), durch welche[n] der Formyl- beziehungsweise Alkylcarbonylrest oder Arylrest, für den $R_3$ stehen kann, substituiert sein kann, [ein] solche[r] mit 1 oder 2, insbesondere 1, Kohlenstoffatom(en) ist beziehungsweise sind.

4.) 5,5-Di-(methyl)-thiazolidin-4(S)-carbonsäurederivate nach Anspruch 1 bis 3, dadurch gekennzeichnet, daß der Alkenylrest, für den $R_1$ stehen kann und/oder der beziehungsweise die Alkylcarbonyloxyrest(e) und/oder Alkylcarbonylrest(e), durch welche[n] der Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest substituiert sein kann, und/oder der beziehungsweise die Alkylcarbonyloxyrest(e), durch welche[n] der Alkyl- oder Arylrest, für den $R_2$ stehen kann, substituiert sein kann und/oder der beziehungsweise die Alkylcarbonyloxyrest(e), durch welche[n] der Alkyl- oder Arylrest, für den $R_3$ stehen kann, substituiert sein kann, und/oder der Alkylcarbonylrest, für den $R_3$ stehen kann, [ein] solche[r] mit 2 oder 3, insbesondere 2, Kohlenstoffatomen ist beziehungsweise sind.

5.) 5,5-Di-(methyl)-thiazolidin-4(S)-carbonsäurederivate nach Anspruch 1 bis 4, dadurch gekennzeichnet, daß der beziehungsweise die Arylrest(e), für den beziehungsweise die $R_2$ und/oder $R_3$ stehen kann beziehungsweise können, [ein] Phenylrest(e) ist beziehungsweise sind.

6.) 5,5-Di-(methyl)-thiazolidin-4(S)-carbonsäurederivate nach Anspruch 1 bis 5, dadurch gekennzeichnet, daß das beziehungsweise die Halogenatom(e), durch welche[s] der Furyl-, Thienyl-, Pyrryl-, Benzofuryl-, Benzothienyl-, Benzopyrryl-, Phenyl-, Pyridyl-, Chinolinyl-, Isochinolinyl- oder Indanylrest, für den $R_1$ stehen kann, der Alkyl- oder Arylrest, für den $R_2$ stehen kann, und/oder der Alkylrest, Formyl- beziehungsweise Alkylcarbonylrest oder Arylrest, für den $R_3$ stehen kann, substituiet sein kann, und/oder das beziehungsweise die Halogenatom(e), durch welche[s] der beziehungsweise die Phenoxyrest(e), durch welche[n] der Formyl- beziehungsweise Alkylcarbonylrest, für den $R_3$ stehen kann, substituiert sein kann beziehungsweise können, substituiert sein kann, Chlor, Brom und/oder Fluor ist beziehungsweise sind.

7.) 5,5-Di-[methyl]-2-[5'-{nitro}-fur-2'-yl]-thiazolidin-4(S)-carbonsäure, 5,5-Di-[methyl]-2-[fur-2'-yl]-thiazolidin-4(S)-carbonsäure, 5,5-Di-[methyl]-2-[thien-2'-yl]-thiazolidin-4(S)-carbonsäure, 5,5-Di-[methyl]-2-[N-(methyl)-pyrr-2'-yl]-thiazolidin-4(S)-carbonsäure, 5,5-Di-(methyl)-2-(pyrid-3'-yl)-thiazolidin-4(S)-carbonsäure, 5,5-Di-(methyl)-2-(pyrid-4'-yl)-thiazolidin-4(S)-carbonsäure, 5,5-Di-(methyl)-2-(benzofur-2'-yl)-thiazolidin-4(S)-carbonsäure, 5,5-Di-(methyl)-2-(indan-5'-yl)-thiazolidin-4(S)-carbonsäure, 5,5-Di-[methyl]-2-[3'-(nitro)-phenyl]-thiazolidin-4(S)-carbonsäure, 5,5-Di[methyl]-2-[4'-(fluor)-phenyl]-thiazolidin-4(S)-carbonsäure, 5,5-Di-[methyl]-2-[(p-methylthio)-phenyl]-thiazolidin-4(S)-carbonsäure, 5,5-Di-[methyl]-2-[2'-(carboxy)-phenyl]-thiazolidin-4(S)-carbonsäure, 5,5-Di-[methyl]-2-[3',4'-di-(methoxy)-2'-(carboxy)-phenyl]-thiazolidin-4(S)-carbonsäure, 5,5-Di-(methyl)-2-(hydroxymethyl)-thiazolidin-4(S)-carbonsäure, 5,5-Di-[methyl]-2-[3',5'-di-(brom)-4'-(hydroxy)-phenyl]-thiazolidin-4(S)-carbonsäure, 5,5-Di-[methyl]-2-[2'-(methyl)-5'-(hydroxymethyl)-3'-(hydroxy)-pyrid-4'-yl]-thiazolidin-4(S)-carbonsäure, 5,5-Di-[methyl]-2-[2',4'-di-(chlor)-phenyl]-thiazolidin-4(S)-carbonsäure, 5,5-Di-(methyl)-2-(p-tolyl)-thiazolidin-4(S)-carbonsäure, 5,5-Di-[methyl]-2-[4'-(hydroxy)-phenyl]-thiazolidin-4(S)-carbonsäure, 5,5-Di-[methyl]-2-[2'-(hydroxy)-phenyl]-thiazolidin-4(S)-carbonsäure, 5,5-Di-[methyl]-2(R)-[p-(fluor)-phenyl]-thiazolidin-4(S)-carbonsäure, 3-[Acetyl]-5,5-di-[methyl]-2(R)-[p-(chlor)-phenyl]-thiazolidin-4(S)-carbonsäure, 3-[Acetyl]-5,5-di-[methyl]-2(S)-[p-(chlor)-phenyl]-thiazolidin-4(S)-carbonsäure, 3-[Acetyl]-5,5-di-[methyl]-2(S)-[2'-(acetoxy)-phenyl]-thiazolidin-4(S)-carbonsäure, 3-[Acetyl]-5,5-di-[methyl]-2(S)-[2',4'-di-(chlor)-phenyl]-thiazolidin-4(S)-carbonsäure, 3-[Acetyl]-5,5-di-[methyl]-2(S)-[p-Tolyl]-thiazolidin-4(S)-carbonsäure, 3-[Acetyl]-5,5-di-[methyl]-2(S)-[phenyl]-thiazolidin-4(S)-carbonsäure, 3-[Acetyl]-5,5-di-[methyl]-2(S)-[4'-(acetoxy)-phenyl]-thiazolidin-4(S)-carbonsäure, 3-[Acetyl]-5,5-di-[methyl]-2(S)-[p-(fluor)-phenyl]-thia-

zolidin-4(S)-carbonsäure, 3-[Acetyl]-5,5-di-[methyl]-2(S)-[5'-(nitro)-fur-2'-
-yl]-thiazolidin-4(S)-carbonsäure, 3-[2'-{p-(chlor-
phenoxy)}-isobutyryl]-5,5-di-[methyl]-2(S)-[phenyl]-
-thiazolidin-4(S)-carbonsäure, 3-[Acetyl]-5,5-di-
-[methyl]-2(R)-[p-(fluor)-phenyl]-thiazolidin-
-4(S)-carbonsäure und 3-(Acetyl)-5,5-di-(methyl)-
-2(R)-(p-{chlor}phenyl)-thiazolidin-4(S)-carbonsäure.

8.) Verfahren zur Herstellung der Verbindungen nach Anspruch 1 bis 7, dadurch gekennzeichnet, daß man, gegebenenfalls an ihrem Stickstoffatom substituierte, ß,ß-Di-(methyl)-cysteine der allgemeinen Formel

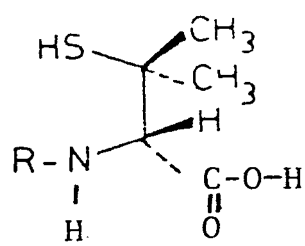

X ,

worin R für Wasserstoff, einen, gegebenenfalls, vorzugsweise durch 1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen,

substituierten, Alkylrest mit 1 bis 4 Kohlenstoffatom(en), einen, gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Alkylthiorest(e) mit 1 bis 4 Kohlenstoffatom(en) und/oder gegebenenfalls durch 1 oder mehr Halogenatom(e) substituierte[n], Phenoxyrest(e), substituierten, Formyl- beziehungsweise Alkylcarbonylrest mit 2 bis 4 Kohlenstoffatomen oder einen, gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n] Alkylcarbonylrest(e) mit 2 bis 4 Kohlenstoffatomen, substituierten, Arylrest, steht, mit Aldehyden der allgemeinen Formeln

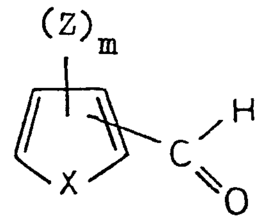 VI,

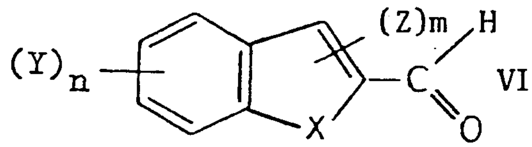 VII,

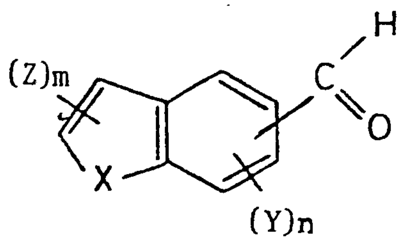 VIII,

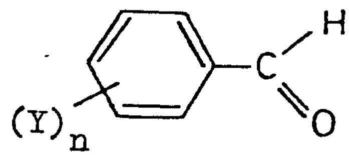 IX,

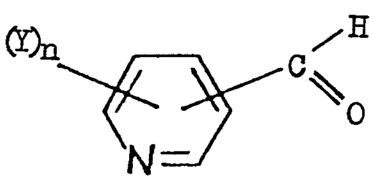 IXa,

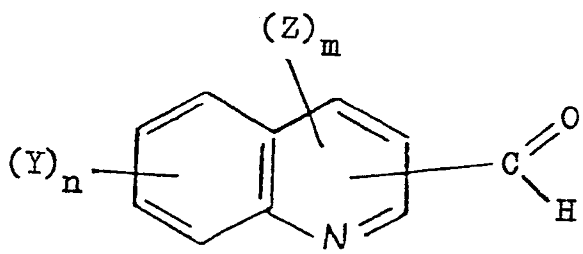 XI,

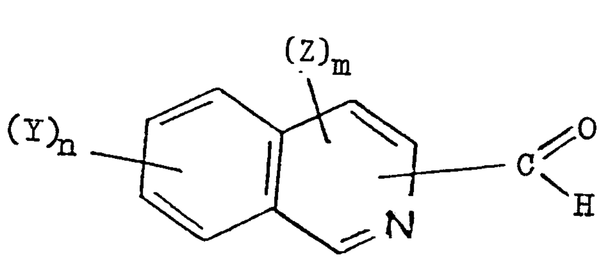 XIa,

0206197

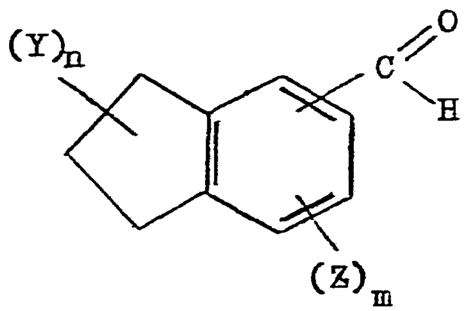

XII,

worin

X für Sauerstoff, Schwefel oder einen Rest der Formel $-\overset{R_0}{\overset{|}{N}}-$ mit $R_0$ = Wasserstoff oder einen organischen Rest, vorzugsweise Alkylrest mit 1 bis 4 Kohlenstoffatom(en), Nitrogruppe, Aminogruppe, Halogenatom, Hydroxygruppe, Hydroxyalkylrest mit 1 bis 4 Kohlenstoffatom(en), Carboxygruppe, Alkoxyrest mit 1 bis 4 Kohlenstoffatom(en), Mercaptorest, Alkylthiorest mit 1 bis 4 Kohlenstoffatom(en), Formyloxy- oder Alkylcarbonyloxyrest mit 2 bis 4 Kohlenstoffatomen oder

Formyl- oder, gegebenenfalls durch 1 oder mehr Hydroxygruppe(n) substituierte[n], Alkylcarbonylrest mit 2 bis 4 Kohlenstoffatomen, steht,

Z für gleiche oder verschiedene organische Rest(e), vorzugsweise Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formvl- und/oder gegebenenfalls durch 1 oder mehr Hydroxygruppe(n) substituierte[n], Alkylcarbonylrest(e) mit 2 bis 4 Kohlenstoffatomen, steht beziehungsweise stehen

Y gleiche oder verschiedene organische Rest(e), vorzugsweise Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit2 bis 4 Kohlenstoffatomen,

bedeutet beziehungsweise bedeuten und

n und m unabhängig voneinander ganze Zahlen von 0 bis 3 bedeuten,

oder mit Aldehyden der allgemeinen Formel

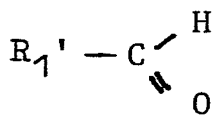

XIII ,

worin

$R_1$' für einen, gegebenenfals durch eine Carboxyl- oder Hydroxygruppe substituierten, Alkylrest mit 1 bis 4 Kohlenstoffatom(en) oder Alkenylrest mit 2 bis 4 Kohlenstoffatomen steht,

umsetzt sowie gegebenenfalls

a) diejenigen 5,5-Di-(methyl)-thiazolidin-4(S)-carbonsäurederivate der allgemeinen Formel I, bei welchen $R_2$ für Wasserstoff steht, in die entsprechenden Alkali- oder Erdalkalisalze überführt oder

b) diejenigen 5,5-Di-(methyl)-thiazolidin-4(S)-carbonsäurederivate der allgemeinen Formel I, bei welchen $R_2$ für Wasserstoff oder ein Alkali- oder Erdalkalimetall steht zu den entsprechenden 5,5-Di-(methyl)-thiazolidin-4(S)-carbonsäurederivaten der allgemeinen Formel I, bei welchen $R_2$ für einen, gegebenenfalls, vorzugsweise durch

1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Alkylrest mit 1 bis 4 Kohlenstoffatom(en) oder, gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Arylrest steht, verestert

und/oder

c) diejenigen 5,5-Di-(methyl)-thiazolidin-4(S)-carbonsäurederivate der allgemeinen Formel I, bei welchen $R_3$ für Wasserstoff steht, mit Alkylhalogeniden oder -estern, Säureanhydriden

oder -halogeniden oder Arylhalogeniden oder -estern zu den entsprechenden 5,5-Di-(methyl)-thiazolidin-4(S)-carbonsäurederivaten der allgemeinen Formel I, bei welchen $R_3$ für einen, gegegebenenfalls, vorzugsweise durch 1 oder mehr Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Alkylrest mit 1 bis 4 Kohlenstoffatom(en), einen gegebenenfalls, vorzugsweise durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en), Alkylthiorest(e) mit 1 bis 4 Kohlenstoffatom(en) und/oder, gegebenenfalls, vorzugsweise durch 1 oder mehr Halogenatom(e) substituierte[n], Phenoxyrest(e) substituierten, Formyl- beziehungsweise Alkylcarbonylrest mit 2 bis 4 Kohlenstoffatomen oder einen, gegebenenfalls durch 1 oder mehr Alkylrest(e) mit 1 bis 4 Kohlenstoffatom(en), Nitrogruppe(n), Aminogruppe(n), Halogenatom(e), Hydroxygruppe(n), Hydroxyalkylrest(e) mit 1 bis 4 Kohlenstoffatom(en), Carboxygruppe(n), Alkoxyrest(e) mit 1 bis 4 Kohlenstoffatom(en),

Mercaptorest(e), Alkylthiorest(e) mit 1 bis 4 Kohlenstoffatom(en), Formyloxy- und/oder Alkylcarbonyloxyrest(e) mit 2 bis 4 Kohlenstoffatomen und/oder Formyl- und/oder, gegebenenfalls durch 1 oder mehr Hydroxygruppe(n) substituierte[n], Alkylcarbonylrest(e) mit 2 bis 4 Kohlenstoffatomen, substituierten, Arylrest steht, N-alkyliert, N-acyliert oder N-aryliert, wobei man gegebenenfalls gleichzeitig eine gegebenenfalls am Substituenten $R_1$ vorhandene Hydroxygruppe acyliert.

9.) Verfahren nach Anspruch 8, dadurch gekennzeichnet, daß, man die Umsetzung der, gegebenenfalls an ihrem Stickstoffatom substituierten, ß,ß-Di-(methyl)--cysteine der allgemeinen Formel X mit den Aldehyden der allgemeinen Formeln VI, VII, VIII, IX, IXa, XI, XIa, XII beziehungsweise XIII in Gegenwart von sauren Katalysatoren, insbesondere Salzsäure, durchführt.

10.) Verfahren nach Anspruch 8 oder 9, dadurch gekennzeichnet, daß man zur Umsetzung der, gegebenenfalls an ihrem Stickstoffatom substituierten, ß,ß-Di-(methyl)-cysteine der allgemeinen Formel X mit den Aldehyden der allgemeinen Formeln VI, VII, VIII, IX, IXa, XI, XIa, XII beziehungsweise XIII als Lösungsmittel Wasser und/oder Methanol oder Pyridin verwendet.

11.) Arzneimittel, gekennzeichnet durch einen Gehalt an 1 oder mehr Verbindung(en) nach Anspruch 1 bis 7 als Wirkstoff(en), zweckmäßig zusammen mit 1 oder mehr üblichen pharmazeutischen Konfektionierungsmittel(n).

Zusammenfassung

Europäisches Patentamt

**EUROPÄISCHER RECHERCHENBERICHT**

0206197
Nummer der Anmeldung

EP 86108186.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | CHEMICAL ABSTRACTS, Band 98, Nr. 15, 11. April 1983, Columbus, Ohio, USA<br>SAMMES, PETER G.; SMITH, STEVEN "On the reduction of ß-lactams with diborane"<br>Seite 603, Spalte 1, Zusammenfassung-Nr. 125 702k<br>& J. Chem. Soc. Chem. Commun. 1982, (20), 1143-4<br>-- | 1 | C 07 D 417/04<br>C 07 D 277/06<br>A 61 K 31/425 |
| X | CHEMICAL ABSTRACTS, Band 102, Nr. 17, 29. April 1985, Columbus, Ohio, USA<br>SAMMES, PETER G.; SMITH, STEVEN; WOOLLEY, GEOFFREY T. "Diborane reduction of penicillins: preparation of 7-deoxopenicillanic acid"<br>Seite 576, Spalte 1, Zusammenfassung-Nr. 148 931z<br>& J. Chem. Soc. Perkin Trans. 1 1984, (11), 2603-10<br>-- | 1 | **RECHERCHIERTE SACHGEBIETE (Int. Cl.4)**<br>C 07 D 417/00<br>C 07 D 277/00 |

Der vorliegende Recherchenbericht wurde für alle Patentansprüche erstellt.

| Recherchenort | Abschlußdatum der Recherche | Prüfer |
|---|---|---|
| WIEN | 22-09-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

| EINSCHLÄGIGE DOKUMENTE | | | EP 86108186.7 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | CHEMICAL ABSTRACTS, Band 91, Nr. 3, 16. Juli 1979, Columbus, Ohio, USA<br>OEHLER, ELISABETH; SCHMIDT, ULRICH "Amino acids and peptides, XXVIII. Dehydro amino acids, XIV. Schiff bases of dehydro amino acids from 2-aryl-4-thiazolidinecarboxylic acids. First synthesis of an N-arylidene-dehydroalanine ester" Seite 707, Spalte 1, Zusammenfassung-Nr. 21 013a<br>& Chem. Ber. 1979, 112(1), 107-15<br>-- | 1 | |
| A | CHEMICAL ABSTRACTS, Band 91, Nr. 25, 17. Dezember 1979, Columbus, Ohio, USA<br>BOGNAR, REZSO; GYOERGYDEAK, ZOLTAN; SZILAGYI, LASZLO; SANDOR, PETER; RADICS, LAJOS "Heterocyclic compounds from sugars. XI. Cyclization of D-penicillamine with 2,3,4,5-tetra-O-acetyl-aldehydro-L-arabinose; carbon-13 NMR spectra of 2-(polyacetoxyalkyl)thiazolidine-4-carboxylic acid derivatives" Seite 714, Spalte 1, Zusammenfassung-Nr. 211 691t<br>& Liebigs Ann. Chem. 1979, (5), 701-7<br>-- | 1 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 22-09-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

0206197

Nummer der Anmeldung

EP 86108186.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | DE - A - 2 221 647 (UNIROYAL)<br>* Formel I *<br>-- | 1 | |
| A | DE - A1 - 2 729 414 (OERIN)<br>* Formel I; Anspruch 5 *<br>-- | 1,11 | |
| A | EP - A1 - 0 045 281 (ZIBA)<br>* Zusammenfassung *<br>---- | 1,11 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 22-09-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82